(19) Europäisches Patentamt
European Patent Office
Office européen des brevets

(11) EP 3 707 244 B1

(12) # EUROPEAN PATENT SPECIFICATION

(45) Date of publication and mention of the grant of the patent:
**03.04.2024 Bulletin 2024/14**

(21) Application number: **18804082.8**

(22) Date of filing: **09.11.2018**

(51) International Patent Classification (IPC):
***C12N 5/077*** *(2010.01)* ***C12N 5/0775*** *(2010.01)*

(52) Cooperative Patent Classification (CPC):
**C12N 5/0656; C12N 5/0662;** C12N 2523/00; C12N 2531/00; C12N 2533/30; C12N 2533/40; C12N 2539/10

(86) International application number:
**PCT/GB2018/053249**

(87) International publication number:
**WO 2019/092434 (16.05.2019 Gazette 2019/20)**

(54) **MACROCARRIER**

MAKROTRÄGER

MACROSUPPORT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.11.2017 GB 201718556**

(43) Date of publication of application:
**16.09.2020 Bulletin 2020/38**

(73) Proprietor: **OXFORD UNIVERSITY INNOVATION LIMITED**
**Oxford OX2 0JB (GB)**

(72) Inventors:
- **NGUYEN THUY BA, Linh**
**Oxford**
**Oxfordshire OX3 7DQ (GB)**
- **YE, Hua**
**Oxford**
**Oxfordshire OX3 7DQ (GB)**
- **CUI, Zhanfeng**
**Oxford**
**Oxfordshire OX3 7DQ (GB)**

(74) Representative: **HGF**
**HGF Limited**
**1 City Walk**
**Leeds LS11 9DX (GB)**

(56) References cited:
**WO-A2-2008/005520 WO-A2-2014/143871**
**US-A1- 2012 156 777 US-A1- 2017 081 638**

- **KIM M R ET AL: "Swelling induced detachment of chondrocytes using RGD-modified poly(N-isopropylacrylamide) hydrogel beads", BIOTECHNOLOGY PROGRESS, AMERICAN CHEMICAL SOCIETY, vol. 18, no. 3, 1 March 2002 (2002-03-01), pages 495-500, XP009122732, ISSN: 8756-7938, DOI: 10.1021/BP020287Z [retrieved on 2002-06-07]**
- **ATSUSHI TAMURA ET AL: "Temperature-responsive poly(-isopropylacrylamide)-grafted microcarriers for large-scale non-invasive harvest of anchorage-dependent cells", BIOMATERIALS, ELSEVIER SCIENCE PUBLISHERS BV., BARKING, GB, vol. 33, no. 15, 31 January 2012 (2012-01-31), pages 3803-3812, XP028468791, ISSN: 0142-9612, DOI: 10.1016/J.BIOMATERIALS.2012.01.060 [retrieved on 2012-02-06]**
- **ZHANG JINNAN ET AL: "Thermo-responsive microcarriers based on poly(N-isopropylacrylamide)", EUROPEAN POLYMER JOURNAL, PERGAMON PRESS LTD. OXFORD, GB, vol. 67, 17 April 2015 (2015-04-17), pages 346-364, XP029230518, ISSN: 0014-3057, DOI: 10.1016/J.EURPOLYMJ.2015.04.013**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 707 244 B1

## Description

The present disclosure relates to a macrocarrier for the propagation of biological cells. The present disclosure also relates to system for the propagation of biological cells, as well as to a process for the propagation of biological cells.

## BACKGROUND

The industrial production of vaccines, enzymes, hormones and cytokines requires cells to be produced on a significant scale. Furthermore, recent advances in stem cell therapy and other cell-based therapeutic treatments often require a scalable quantity of cells to be produced.

Cells may be propagated in a bioreactor, where cells are grown on suspended microcarriers in a culture medium. The microcarriers act as supporting substrates to which cells are anchored during cell culturing process. Microcarriers are relatively small and typically range from 125 to 250 $\mu$m in size. Accordingly, they are easily suspended in culturing media and have a relatively high surface area to volume ratio for supporting cell attachment and growth. Following the culture stage, the anchored cells require separation from the microcarrier beads in order to be recovered.

There are a range of methods for recovering cells from microcarriers. For example, the cells may be recovered by enzymatic digestion, for example, using trypsin, accutase or collagenase. However, while such methods may be effective for separating the cells from the microcarriers, the treatment can sometimes have a negative effect on the physiology of the cells produced. This can have a negative effect on the quality and viability of the recovered cells.

Thermoresponsive polymers are polymers that show a significant change in properties upon a small change in temperature. An example of such a polymer is poly-N-isopropylacrylamide (PNIPAAm). Depending on the temperature, PNIPAAm can change from a hydrophilic, random coil conformation to a hydrophobic, collapsed globular conformation. Biological cells are typically attracted to the hydrophobic surface and repelled from the hydrophilic surface. Accordingly, the polymer can be used to provide a cell-receiving surface e.g. above a threshold temperature and a cell-releasing surface as the temperature falls. As such, cells may be anchored to the polymer above a threshold temperature, until such time as separation and isolation are required, whereby the temperature is lowered, and the cells detach. Unlike enzymatic treatments, this method of detachment may reduce the risk of damage to the physiology of the cell. In Cell Transplantation, Vol. 19, pp. 1123-1132, 2010, the thermosensitive polymer, PNIPAAm, is conjugated onto microcarrier beads having a diameter of 170 to 380 microns.

M. R. Kim et al., Biotechnol. Prog., Vol. 18, pp. 495-500, 2002, describes poly(N-isopropylacrylamide) hydrogel beads conjugated with a cell adhesive peptide, GRGDY.

WO 2014/143871 A2 describes a scaffold for culturing cells, which comprises a thermoresponsive polymer, such as N-isopropylacrylamide (NIPAAm).

US 2012/156777 A1 describes a carrier for growing adherent cells, which comprises one or more outer surfaces; and one or more structured indentations on one or more of the outer surfaces, wherein the carrier has a length at least about 0.2 mm, a width at least about 0.2 mm, and a height in a range from about 0.05 mm to 1.2 mm and each of the structured indentations has a major axis in a range from about 0.1 mm to 0.5 mm, a minor axis in a range from about 0.1 mm to 0.5 mm and a depth in a range from about 0.025 mm to about 0.5 mm.

WO 2008/005520 A2 describes a cell culture substrate comprising: a support coated with a temperature-responsive polymer or copolymer; wherein the support is a cell-compatible microcarrier; wherein the temperature-responsive polymer binds cells at a first temperature compatible with cell proliferation and releases cells at a second temperature that is compatible with cell viability.

US 2017/081638 A1 describes a method comprises culturing stem cells in a bioreactor system in the presence of a thermally responsive microcarrier.

## SUMMARY OF THE INVENTION

The invention is set out in the appended set of claims.

## BRIEF DESCRIPTION OF THE FIGURES

Aspects of the present disclosure are shown schematically, by way of example only, in the accompanying drawings, in which:

Figure 1 is a schematic diagram of thermo-responsive polymer grafting onto the surfaces of PCL and the temperature-dependent effect of cell attachment to and detachment from the grafted surface;

Figures 2a and 2b show FTIR and XPS spectra showing the conjugation of PNIPAAm-$NH_2$ to the surface of PCL

beads;

Figure 3 shows cell proliferation on macrocarrier surfaces;

Figures 4a and 4b show cell detachment and cell viability data of cells detached from macrocarrier surfaces (i.e. trypsinization vs. reduced temperature comparison of cell detachment ratio and viability);

Figure 5 shows recovered cell proliferation comparisons between different cells detached by reduced temperature and trypsinization;

Figure 6 is a Western blot analysis of proteins collected from cells grown on tissue culture plates and thermoresponsive macrocarriers; the cells were detached by trypsin-EDTA and by reducing the temperature;

Figure 7 is a diagram containing a series of images that show the preparation of PCL pellets;

Figure 8 shows SEM images of PCL beads that were prepared according to the method described in Example 2 and SEM images of PCL beads that are commercially available;

Figure 9 shows an SEM image of PCL beads and a series of histograms showing the size distribution of the beads;

Figure 10 show FTIR and XPS spectra of PCL beads that were prepared according to the method described in Example 2 and PCL beads that are commercially available;

Figure 11 shows SEM images and EDS spectra of porous PCL beads and PCL-PNIPAAm macrocarriers;

Figure 12 shows an SEM image of a pore on the surface of a PCL-PNIPAAm macrocarrier;

Figure 13 is a series of images showing the cell proliferation of MSC seeded on PCL and PCL-PNIPAAm macrocarriers as observed by a fluorescence microscope in low magnification;

Figure 14 is a series of images showing the cell proliferation of MSC seeded on PCL and PCL-PNIPAAm macrocarriers as observed by a fluorescence microscope in high magnification;

Figures 15 and 16 each show histograms that illustrate the cell proliferation on various macrocarrier surfaces over several days; and

Figure 17 shows images of MSC detached from PCL-PNIPAAm.

## DETAILED DESCRIPTION

In one aspect of the present invention, there is provided a macrocarrier for the propagation of biological cells. The macrocarrier comprises substrate particles that are coated with a thermoresponsive polymer that provides the macrocarrier with a cell-receiving surface and responding to a change in temperature to release cells from the macrocarrier, wherein: the cell-receiving surface is porous; each substrate particle has a spherical, ellipsoid, ovoid or ring shape; and at least 50% of the substrate particles have a particle size of at least 1 mm, wherein the particle size is the largest linear dimension across the substrate particle.

In another aspect, there is provided a system for the propagation of biological cells. The system comprises a bio-reactor and a macrocarrier as described in the present disclosure.

In yet another aspect, there is provided a process for the propagation of biological cells. The process comprises contacting biological cells with a macrocarrier of the present disclosure in a cell culturing medium; subjecting the macrocarrier to a temperature at which the thermoresponsive polymer presents a cell-receiving surface and propagating the cells on the macrocarrier; and subsequently altering the temperature of the macrocarrier to release any propagated cells from the macrocarrier.

Advantageously, a significant proportion of the substrate particles have a particle size of at least 1 mm. In contrast to microcarriers employed in prior art bioreactor systems that are typically 125 to 250 $\mu$m in size, a larger particle size can present biological cells with a 'flatter' surface upon which to adhere. It is believed that this flatter surface results in the adhered cells being less torsional constrained, or twisted, and subjected to less shearing force or mechanical stress during agitation in the bioreactor. As a result, the cells may be exposed to a gentler, more uniform, and optimal

growing environment. This can help to improve the quality (e.g. viability and health) of the cells produced. Moreover, because the macrocarriers of the present disclosure are coated with a thermoresponsive polymer, cells can be released from the macrocarrier by changing the surrounding temperature. This allows the cells to be recovered without the need, for example, of enzymatic treatments that can present an increased risk of cell damage.

The larger particle size may also aid cell collection and separation from the macrocarrier when compared to a microcarrier. It is thought that a cell will require more energy to detach from a microcarrier in comparison to a macrocarrier, due to differences in the radii and surface area curvatures of the carriers.

On microcarriers, cells tend to clump and grow in aggregate when they are cultured in a bioreactor. In many clinical, biotechnological and tissue engineering settings, it is necessary to produce discrete cells and the production of clumps of cells can be problematic. When cells are cultured on macrocarriers in a bioreactor they do not, in general, aggregate. Cells attached to a macrocarrier are also less likely to suffer damage in a bioreactor compared to cells attached to a microcarrier.

As noted above, at least 50% of the substrate particles have a particle size of at least 1 mm. In some examples, at least 70%, preferably at least 80 or 90% of the substrate particles have a particle size of at least 1 mm. By ensuring that a significant proportion of the substrate particles are large, advantage can be taken of the "flatter" support surface. As described above, this allows the cells to be cultivated under gentler conditions, reducing the shear forces to which they are exposed during propagation within the bioreactor. This, in turn, can result in e.g. healthier and more viable cells.

It is preferred that the substrate particles have a relatively narrow size distribution. For example, the substrate particles may be sufficiently large to present a desirable surface for cell attachment and propagation but, at the same time, be sufficiently small to be suspended in the culture medium of a bioreactor. For instance, at least 50% of the substrate particles may have a particle size of 1 mm to 10 mm. In other examples, at least 50% of the substrate particles have a particle size of 2 mm to 6 mm. In other examples, at least 50% of the substrate particles have a particle size of 3 mm to 5 mm. In other examples, at least 70% of the substrate particles have a particle size of 1 mm to 10 mm. In other examples, at least 70% of the substrate particles have a particle size of 2 mm to 6 mm. In other examples, at least 70% of the substrate particles have a particle size of 3 mm to 5 mm. In other examples, at least 80% or 90% of the substrate particles have a particle size of 1 mm to 10 mm. In other examples, at least 80% or 90% of the substrate particles have a particle size of 2 mm to 6 mm. In other examples, at least 80% or 90% of the substrate particles have a particle size of 3 mm to 5 mm. In a preferred embodiment, 95 to 100% of the substrate particles have a particle size of 1 to 10 mm, preferably 2 to 6 mm, and more preferably 3 to 5 mm.

In principle, any suitable substrate particle may be used in the macrocarrier of the present disclosure. The substrate has a substantially spherical, ellipsoid, ovoid, or ring shape. In one example, the substrate particle may take the form of a bead. The bead has a substantially spherical, elliptical, ovoid, or ring. Where the substrate particles are non-spherical, their particle size refers to the largest linear dimension across the substrate particle. For example, where the substrate particles are ellipsoid, the particle size refers to the length of the major axis of the ellipsoid. It may be preferable that the substrate particle(s) is/are substantially spherical.

The substrate particle may be formed of any suitable material. Preferably, the material is a biocompatible material, for example, a biocompatible polymer. In some examples, the material is biocompatible according to ISO 10993. Suitable materials include polysaccharide, protein, glass, polystyrene, polyester, polyolefin, silica, silicone, polyacrylamide and polyacrylate. Suitable polysaccharides include dextran (e.g. diethylaminoethanol (DEAE) - dextran), chitosan and alginate. A suitable protein may be collagen. An example of a suitable polyacrylate is poly(2-hydroxyethyl methacrylate). Preferably, the substrate particle comprises a polyester.

In a preferred embodiment, the substrate particle comprises a polyester selected from the group consisting of polylactic acid ("PLA"), polyglycolic acid ("PGA"), polycaprolactone ("PCL"), polybutyrolactone ("PBL"), polyvalerolactone ("PVL"), polyhydroxybutyrate ("PHB"), poly (3-hydroxy valerate), poly(ethylene succinate) ("PESu"), and poly(butylene succinate) ("PBSu"). In a more preferred embodiment, the polyester is PCL.

Any suitable thermoresponsive polymer may be used in the macrocarrier of the present disclosure. A thermoresponsive polymer, also referred to as a thermosensitive polymer, is a polymer that shows a significant change in properties upon a small change in temperature. In the present disclosure, the thermoresponsive polymer that is used to coat the substrate particles is capable of providing the macrocarrier with a cell-receiving surface onto which cells can be attached. The thermoresponsive polymer, however, responds to a change in temperature to release cells from the macrocarrier.

In some examples, the thermoresponsive polymer is one that changes its morphology upon exposure to a change in temperature. The thermoresponsive polymer may be made up of hydrophobic and hydrophilic parts that change their orientation upon a change in temperature, so as to present a hydrophobic surface or a hydrophilic surface to the external environment. For instance, in one embodiment, the thermoresponsive polymer changes from a hydrophilic, random coil conformation to a hydrophobic, collapsed globular conformation in response to a change in temperature. The hydrophobic surface may be presented to allow, for example, immobilisation or attachment of cells. On the other hand, the hydrophilic surface may repel the attached cells, allowing them to be released from the polymer surface.

In some examples, the threshold temperature is at or below a temperature suitable for culturing, propagating,

or differentiating cells. In another example, the threshold temperature is above a temperature suitable for detaching cells but maintaining quality and viability of the cells. In another example, the temperature range difference between the thermoresponsive polymer providing a cell-receiving surface and a cell-repelling surface is optimised so as to maximise cell tethering for culturing, propagation or differentiation, but maximise cell detaching once the culturing, propagation or differentiation step is complete. In another example, the difference in temperature between that at which cells tether and that at which cells detach should be minimised so as to reduce the possibility of cold shock, or low-temperature stress, on the propagated cells.

In some examples, the thermoresponsive polymer provides the macrocarrier with a cell-receiving (e.g. hydrophobic) surface above a threshold temperature and releases cells (e.g. by presenting a hydrophilic surface) from the macrocarrier below the threshold temperature. Accordingly, the macrocarrier may be maintained above the threshold temperature (e.g. in a bioreactor) to allow attachment of cells and facilitate their propagation. Subsequently, the temperature may be reduced below the threshold temperature to release the propagated cells from the macrocarrier. This can allow convenient cell recovery.

In some examples, the threshold temperature may be a temperature of between 20°C and 40°C. In some examples, the threshold temperature is a temperature of between 30°C and 40°C. In some examples, the threshold temperature is a temperature of between 30°C to 37°C. In some examples, the threshold temperature is about 32°C.

In some examples, the macrocarrier may be maintained above the threshold temperature at a temperature that facilitates or optimises cell propagation and growth in order to allow for cell attachment and propagation on the macrocarrier. This temperature may be, for example, about 34 to 39 °C, preferably, at about 37 °C. Subsequently, the macrocarrier may be cooled, for instance, to below the threshold temperature of the thermoresponsive polymer to release the cells. This macrocarrier may be cooled to a temperature below 33 °C, for instance, below 32 °C. In some examples, the macrocarrier may be cooled to 30 °C to release the propagated cells.

A number of thermoresponsive polymers may be used to coat the macrocarrier, such as a polymer selected from the group consisting of poly(N-isopropyl acrylamide) ("PNIPAAm"), poly(butylmethacrylate) ("PBMA"), poly(D,L-lactide) ("PDDLA"), poly(N,N-diethylacrylamide) ("PDEAAm"), poly(N-vinylcaprolactam) ("PNVCL"), poly[2-(dimethylamino)ethyl methacrylate] ("PDMAEMA"), poly(ethylene oxide-b-propylene oxide-b-ethylene oxide) ("PEO-PPO-PEO"), poly(ethylene glycol-b-(DL-lactic acid-co-glycolic acid)-b-ethylene glycol) ("PEG-PLGA-PEG"), poly(methyl 2-propionamidoacrylate) ("PMPA"), poly([DL-lactic acid-co-glycolic acid]-b-ethylene glycol-b-[DL-lactic acid-co-glycolic acid]) ("PLGA-PEG-PLGA"). In an example, the thermoresponsive polymer is PNIPAAm. Depending on the temperature, PNIPAAm can change from a hydrophilic, random coil conformation to a hydrophobic, collapsed globular conformation.

The thermoresponsive polymer may be coated onto the substrate particles using any suitable method. The substrate particles may be at least partially coated with the thermoresponsive polymer.

In some examples, the thermoresponsive polymer may be covalently attached to the substrate particle. The thermoresponsive polymer may be suitably functionalised with a group capable of forming a covalent bond to a suitably functionalised substrate particle. In some examples, the thermoresponsive polymer may be functionalised with more than one type of functional group. In some examples, the substrate particle may be functionalised with more than one type of functional group. In some examples, more than one thermoresponsive polymer is attached to one substrate particle. In some examples, the thermoresponsive polymer is functionalised with an amino group and the substrate particle is functionalised with an acid group, thereby allowing the formation of an amide bond as the covalent linkage. In another example, the thermoresponsive polymer could be functionalised with the acid group and the substrate particle functionalised with the amino group, thereby allowing the reverse amide bond to be formed as the covalent linkage. In some examples, the thermoresponsive polymer is PNIPAAm that is functionalised with an amino group, and the substrate particle is PCL that is functionalised with an acid group. Other complementary groups on PCL and PNIPAAm capable of forming amide bonds, such as for example esters, acyl halides, and anhydrides, are encompassed within the scope of the disclosure, as are complementary groups capable of forming covalent bonds other than amide bonds. Alternative complementary functional groups for coupling the substrate particle to the thermosensitive polymer will be apparent to the skilled person as means to covalently link these moieties other than through an amide bond. Examples of such coupling groups include hydroxyl, thiol, halo, sulphonyl, aldehyde, epoxy, and the like.

Typically, the substrate particle does not comprise a thermoresponsive polymer. Thus, the substrate particle may not be made of a thermoresponsive polymer.

The substrate particles may be solid or porous. Where porous particles are used, oxygen in the culture medium may diffuse through the particles towards any cells attached to the particles surface. Porous particles may have a lower density than non-porous particles made of the same material that occupy the same volume. When the substrate particles have a relatively low density, the resulting macrocarriers may float in the bioreactor or may be easily stirred or swirled within the bioreactor.

The substrate particles may have a relative density with respect to water of less than 1 (i.e. at atmospheric pressure and a temperature of 20 °C), such as < 0.85, particularly < 0.70.

The bulk density of the substrate particles is typically less than the true density of the solid material from which

the substrate particles is made. The term "bulk density" as used herein refers to the mass of one or more substrate particles divided by the total volume that they occupy. The bulk density measurement includes the volume of the solid material from which the particles are made and any pores, whether open or closed. The term "true density" as used herein refers to the density of the solid material from which the particles are made. The measurement of true density therefore excludes the volume of any open and closed pores.

The substrate particles may have a ratio of bulk density to true density of < 0.80:1, such as < 0.70:1, particularly < 0.60:1. For the avoidance of doubt, the bulk density and true density measurements should be determined at atmospheric pressure and a temperature of 20 °C.

The surfaces of the substrate particles may be porous. The porous surfaces of the substrate particles may be retained after coating with a thermoresponsive polymer.

The macrocarrier of the present disclosure has a cell-receiving surface that is porous.

The pores on the surface of the macrocarrier can absorb and retain both nutrients and medium, which are needed for cell growth. The close proximity of nutrients and medium may facilitate the rapid propagation of cells, such that a significant number of high quality cells can be produced over a shorter time. For example, the stationary phase for cell growth may be reached more quickly using macrocarriers having a porous surface compared to macrocarriers having a non-porous and dense surface.

In comparison to macrocarriers having a non-porous surface, the surface of porous macrocarriers is relatively rough. This relatively rough surface can aid cell proliferation because cells adhere to rough surfaces better than they adhere to smooth surfaces.

When a surface of the macrocarrier, such as the cell-receiving surface, is porous, then the pores may have a size of ≤ 20 μm, such as ≤ 10 μm, particularly ≤ 5 μm. The pores typically have a size that is less than the size of a grown cell. Pore size may be determined by SEM imaging.

The macrocarrier of the present disclosure may be used to propagate any biological cell. In other words, the cells may be any cell capable of adhering and growing on the surface of the macrocarrier. Examples include mammalian as well as hybrid cell lines and tumour-based cells. Preferably, the cells are mammalian cells. The mammalian cells may be cultured for the following tissue types: for example, bone marrow, carcinoma, conjunctiva, cornea, endothelium, epithelium, fibroblast, fibrosarcoma, heart, hepatoma, liver, lung, macrophage, melanoma, muscle, neuroblastoma, osteosarcoma, ovary, pancreas, pituitary, rhabdomyosarcoma, synovial fluid, thyroid, and the like.

As an example, the following cell types may be propagated on macrocarriers of the present disclosure: bovine (endothelial, kidney, muscle), canine (MDCK), chicken (embryo, fibroblast, muscle, myoblasts), fish (RTG-2, AS, CHSE-214), guinea pig (GPK), hamster (BHK, BHK21, BHK21 C13, CHO, CHO-K1, CHO-recombinant)), human (adenocarcinoma, amniotic, bladder cancer, breast cancer, endothelial, fibroblast, FS, FS-4, HeLa, HEL 299, IMR, K-562, KB, kidney, lymphoblastoid, lymphocyte, MCF-7, monocyte, MRC-5, osteosarcoma, pancreas), monkey (BSC-1, CV-1, kidney, LLC-MK, Vero), mouse (fibroblast, L-929, macrophage, mesenchyme), pig (endothelial, testicular, thyroid), rat (epithelium, myoblast, pancreas, pituitary), and turkey (pituitary). In addition, cells that are cultures on macrocarriers can be used as substrates for the production of vaccines, vectors, natural and recombinant proteins, monoclonal antibodies, and other biological products.

In another example, a composition is provided of a plurality of cells tethered to a macrocarrier. Some examples are mesenchymal stem cells ("MSCs"), human dermal fibroblasts ("HDFs"), human umbilical vein endothelial cells (HUVEC) and neuroblastoma Sy5y cells.

In another example, a system is provided for biochemical engineering, wherein the proliferated cells remain tethered to the macrocarrier for further culturing so as to generate a variety of downstream products, prior to cell release. Such downstream products could include for example cell therapy products from bioprocessed stem cells, recombinant protein production, antibody and virus generation, and gene amplification, to name a few possible biomanufacturing applications. Conditions for fibroblast cell culture systems for synthesizing extracellular matrix and collagen could be utilised in the tethered fibroblast macrocarrier system herein.

## Examples

### <u>Example 1</u>

### <u>Materials and Methods</u>

#### Materials

All materials were purchased from Sigma-Aldrich (UK) and used as received. The materials were: polycaprolactone pellets (PCL, Mn 80,000), sodium hydroxide (NaOH), 1-ethyl-3-[3-dimethylaminopropyl]carbodiimide hydrochloride (EDC), Sulfo-N-hydroxysuccinimide (Sulfo-NHS), morpholinoethanesulphonic acid (MES) and poly (N-isopropylacrylamide) amine terminated average Mn 2500 (T) (PNIPAAm-$NH_2$). Deionised water (DI water) used in this study was obtained from an ultrapure water purification system (Elix®, Millipore).

**Preparation of PCL-PNIPAAm**

The PCL pellets were immersed in NaOH 1M solution for 1h with constant shaking to obtain carboxylate ions PCL-$COO^-$, then they were rinsed with for autoclaved deionized water (DI) several times. PCL-PNIPAAm macro-carriers were synthesized by conjugating PCL-$COO^-$ pellets with PNIPAAm-$NH_2$ through an amidation reaction. The PCL-$COO^-$ pellets were activated by 1-ethyl-3-[3-dimethylaminopropyl]carbodiimide hydrochloride (EDC, 0.12M) and Sulfo-N-hydroxysuccinimide (Sulfo-NHS, 0.06M) in 0.05M morpholinoethanesulphonic acid (MES, 0.05M) buffer solution (pH 6) for 3 h at room temperature. PNIPAAm-$NH_2$ was added to the activated PCL-$COO^-$ solution and gently shaken at 4 °C overnight. The solution containing PCL-PNIPAAm macro-carriers was centrifuged at 1500 rpm for 10 min, washed five times with deionized distilled water, and lyophilized for 2 days.

The reaction scheme is shown schematically below:

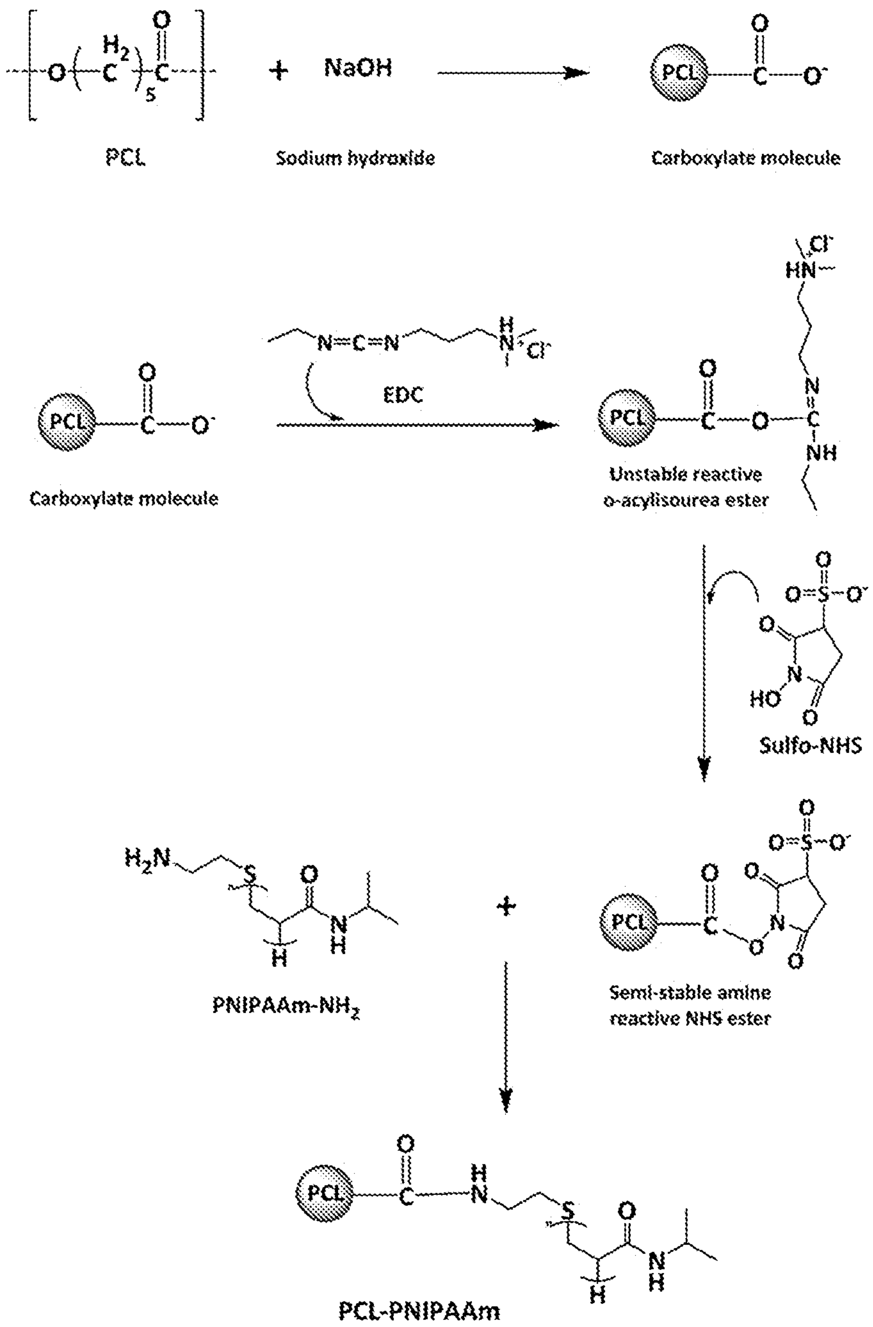

**Characterizations and measurements**

Fourier transform infrared (FTIR) spectra were recorded using an FTIR spectrometer (Bruker, Tensor 27) equipped with attenuated total reflectance (ATR, Pike). Before collecting sample spectra, the background spectrum was collected by measuring the response of the spectrometer without a sample.

X-ray photoelectron spectroscopy (XPS) spectra for PCL and PCL-PNIPAAm were obtained base pressure $1 \times 10^{-9}$ torr, variable aperture 3-10 mm and data analysed using CasaXPS peak fitting software.

**Cell culture on thermo-responsive macrocarriers**

Human dermal fibroblast cells (HDF, ThermoFisher Scientific) were cultured in Dulbecco's modified Eagle's medium (DMEM 4.5 mg/l of glucose; Gibco BRL, Gaithersburg, MD, USA) supplemented with 10% (v/v) fetal bovine serum (FBS; Gibco BRL) and 1% (v/v) penicillin-streptomycin (PS; Gibco BRL) and Green Fluorescence Protein (GFP) was cloned into Mesenchymal stem cells (MSC, kindly provided from Department of Paediatrics and Adolescent Medicine, LKS Faculty of Medicine, The University of Hong Kong). Siliconized (Sigmacote® treated) glass bottles were prepared prior to use to prevent cells adhering to the bottle walls. PCL and PCL-PNIPAAm macrocarriers were washed with phosphate buffer saline (PBS) for 15 min and incubated in DMEM at 37 °C, overnight.

*Cell viability, cytotoxicity and proliferation assessment*

Cell viability, cytotoxicity and proliferation were determined by CCK-8 assay (Sigma). To measure the efficiency of cell attachment to macrocarriers within 24 h of culture, the macrocarrier-free supernatant was carefully removed and the number of cells in the supernatant was determined with a hemocytometer. The number of cells attached to macrocarriers was calculated by subtracting the number of cells in the supernatant from the total cell number at inoculation. The attachment yield was calculated as follows:

Attachment yield (%) = (number of cells attached to macrocarriers/total number of cells number at inoculation) x 100.

*Cell detachment from macrocarriers*

After 1 day of suspension culture, the temperature of the culture medium was reduced from 37°C to 30°C by using an incubator and HDF cultured on two types of macrocarriers were incubated for 40 min at 30°C. The number of detached cells in the macrocarrier-free supernatant was counted with a hemocytometer.

Cell detachment ratio = [number of detached cells]/[total number of attached cells on macro-carriers before detachment] x 100.

*Extra cellular matrix (ECM) protein expression*

ECM protein expression of HDF was analysed by western blot analysis. To detect the fibronectin, laminin and collagen I on the detached cells, anti-fibronectin (1:200, Santa Cruz, Biotechnology, CA, USA), anti-laminin (1:200, Santa Cruz, Biotechnology, CA, USA) and anti-collagen I (1:50, Santa Cruz, Biotechnology, CA, USA) anti-bodies were used.

For western blot analysis: HDF were rinsed and harvested in lysis buffer (RIPA, Millipore, Milford, MA, USA), vortexed on ice 5 times within 20 min and centrifuged for 10 min at 13,000 rpm at 4 °C. Anti-fibronectin, anti-laminin and anti-collagen type I antibodies were used as proteins for the analysis. Anti-beta actin antibody was used as housekeeping control. The immunocomplexes were visualized using enhanced chemiluminescence reagent.

**Statistical Analysis**

All quantitative data were expressed as mean ± SD. Statistical analysis was performed with two-way analysis of variance (ANOVA) with Tukey's honestly significant difference post hoc test. All analyses were carried out using GraphPad Prism 6 with a value of $p < 0.05$ was considered statistically significant.

## Results and Discussion

### *Characterization of PNIPAAm grafted PCL macrocarriers*

Figure 1 shows a schematic diagram of thermo-responsive polymer grafting onto the surfaces of PCL and the temperature-dependent effect of cell attachment to and detachment from the grafted surface. Lowering the temperature as low as 30 °C cause cellular detachment due to the hydrophilic conformation of PNIPAAm. At 37 °C the conformation of PNIPAAm is globular conformation which provides a hydrophobic surface on the PCL macrocarrier. When the temperature is dropped down to 30 °C, the conformation changes to randomised coil causing the surface to become hydrophilic in nature. A hydrophobic surface attracts cells and a hydrophilic surface repels the cells.

In order to graft the thermoresponsive polymer onto the surfaces of PCL beads, PNIPAAm-$NH_2$ polymers were conjugated with PCL beads through amidation between the carboxylate molecule on PCL beads' surface and the amine end group of PNIPAAm-$NH_2$. PCL pellets were initially treated with NaOH, which cause the base hydrolysis of esters bonds in PCL creating carboxylate ions. This carboxyl functional groups on PCL beads can be activated by EDC and NHS to form succinimide esters, which in turn spontaneously react with the amine groups on the PNIPAAm-$NH_2$. The reaction of carboxylate with EDC creating an unstable reactive O-acylisourea ester. Sulfo-NHS is then added to stabilize the intermediate, which converts the unstable O-acylisourea into an amine-reactive NHS ester. This ester will react with the amino end of the PNIPAAm-$NH_2$ to form a stable covalent amide bond between the PCL beads and the PNIPAAm-$NH_2$. The scheme of conjugation of PNIPAAm-$NH_2$ to PCL is shown in figure 1.

FTIR spectroscopy was used to confirm the conjugation of PNIPAAm-$NH_2$ to the surface of PCL beads. The FTIR spectrum in figure 2a shows the appearance of several new peaks due to PNIPAAm-$NH_2$ introduction onto PCL-COOH. The wide peak between 3550 and 3200 $cm^{-1}$ belongs to the N-H stretching of the modified PCL beads. The increasing of peak at 2940 $cm^{-1}$ is attributed to the vibration of aliphatic groups $(-CH_2-)_n$ of the copolymer. The increasing of intensity peak at 1647 $cm^{-1}$ could be the sign of amide I bond, arising from C=O stretching and little C-N stretching of PNIPAAm. The peaks at 1565 $cm^{-1}$ corresponding to amide II bond, arising from N-H bending and C-N stretching of PNIPAAm. This suggested that the conjugation of the PNIPAAm on PCL beads was successful.

XPS determines the chemical composition of a surfaces top several nanometres. The appearance of a new N1s signal with binding energy at 400 eV after PNIPAAm grafting in the wide scan XPS spectra in figure 2b were indicative of successful grafting of PNIPAAm on the PCL macrocarriers surface. The N1s core-level spectra from PCL-P was curve-fitted with peak at 399.4 eV attributable to the amino group ($-NH_2$). Detection of N and C from C=O bonds means that PNIPAAm-$NH_2$ is present on the surface of PCL beads.

### *Cell viability, cytotoxicity and proliferation assessment*

In order to assess whether the grafting material and other chemical reactions of the grafting procedure caused any adverse effect on cell growth and viability we used CCK-8 assay for viability up to 7 days. Although compared to the control both PCL and PCL-PNIPAAm surfaces showed reduced viability, a consistent increase in cell proliferation on both surfaces was depicted as shown in figure 3. The results showed that both HDF cells and MSCs survived and proliferated on both PCL and PCL-P (PCL-PNIPAAm) and tissue culture plate as control (TCP CON) surfaces for 1, 3 and 7 days (a). At day 7, a significant increase in OD was observed in both cell types compared to day 1 and 3 as well as a both cells survived higher on the surfaces of PCL-P than the surfaces of PCL (* $p < 0.05$; ** $p < 0.01$; *** $p< 0.001$; ns: no significant different). In addition, a slightly healthier proliferation rate was observed in grafted surfaces than the non-grafted PCL (* $p < 0.05$ at day 7).

Altogether these results suggested that PNIPAAm grafting onto PCL surfaces was risk-free and thus provided a valuable tool for recovering large-scale cellular collections.

### *Cell detachment from macrocarriers*

In order to ascertain efficiency of cell detachment by lowering the temperature to 30°C we compared the recovered cells in the reduced temperature condition with trypsinization conditions.

Figure 4a shows that both HDF cells and MSCs in reduced temperature conditions (30 °C) showed a significantly higher cell-detachment ratio from PCL-PNIPAAm surfaces than from PCL only surfaces. Trypsinization did not show a significant difference in the extent of cell detachment between the surfaces but this technique showed more detachment of cells from the surfaces of both PCL and PCL-PNIPAAm surfaces. Figure 4b shows that a higher cell viability rate was observed in temperature dependent cell recovery technique than by trypsinization. Both HDF and MSCs had higher survival when recovered from PCL-PNIPAAm than by trypsinization (* $p < 0.05$; ** $p < 0.01$; *** $p< 0.001$; ns: no significant difference).

Figures 4a and 4b show that more than 70% of the cells were detached from PNIPAAm-grafted PCL surfaces

by simply lowering the temperature. In contrast, trypsinization had a higher detachment rate than the reduced temperature technique of thermoresponsive polymer. This was not surprising; other research groups have also reported higher detachment rates with enzymatic digestion than with the thermal reduction technique. However, the physiological damage caused by trypsin or other enzymes is the major reason why researchers wish to avoid enzymatic digestion in clinical applications.

***Recovered cell proliferation after detachment from PCL-PNIPAAm***

To demonstrate the propagation and proliferation into the immediate cellular passage of the harvested and recovered cells, a comparative viability assay was conducted for 1, 3 and 7 days between trypsin treated and recovered cells and reduced temperature and recovered cells (figures 5a and 5b). Both HDF cells (figure 5a) and MSCs (figure 5b) from PCL-PNIPAAm surfaces were recovered, collected and grown at 1, 3 and 7 days in culture media. After recovering the cells either by using trypsin-EDTA or reduced temperature, equal numbers of cells were seeded for proliferations. CCK-8 studies showed when both cell types were recovered from PCL-PNIPAAm surfaces using reduced temperature, cell growths were exponential and significant over time. However, when collected by trypsinization, cell growths were non-exponential and insignificant over time (* $p < 0.05$; ** $p < 0.01$; *** $p< 0.001$; **** $p < 0.0001$; ns: no significant difference). The result showed a logarithmic growth with significant increase in cell numbers in both cell types collected from PCL-PNIPAAm surfaces by reducing temperature. This result clearly indicated that cell recovery process involving reduced temperature process was more efficient than the Trypsin-EDTA recovery process.

***ECMs on cells detached from PCL-PNIPAAm macrocarriers***

The fate of the expression of ECM proteins on cells detached from PCL-PNIPAAm by either trypsin or reduces temperature was observed by immunoblotting. In this study, we seeded HDF on tissue culture plate and PCL-PNIPAAm, incubated for 7 days, and then collected them either trypsin-EDTA treatment or simply by reducing temperature. Cells were then immunostained or subjected for total protein collection for Western blotting. Three major structural ECM proteins such as Fibronectin (FN), Laminin (LM) and Collagen type I (Col I), which are the most abundant and important ECM proteins found in cells/tissues, play various roles in foetal development, tissue repair and angiogenesis. Because of their 'glue-like' properties, these proteins usually play a role in cell attachment on the surfaces. Here we studied these proteins to see whether the process employed to cell recovery affected their expression or not.

The expression patterns of Fibronectin, Laminin and Collagen I in recovered HDF cells (after detachment and collection) were analysed by Western blot as shown in figure 6. Total proteins were collected from cells grown in tissue culture plates by harvesting the total cells either using trypsin-EDTA (Control-TE) or by scraping the monolayer of cells by a scraper (Control-Scraper). Cells were also grown on PCL-PNIPAAm beads and harvested either by trypsin-EDTA (PCL-P-TE) by reducing the temperature (PCL-P-Reduce). Antigen against laminin, Fibronectin and Col 1 were used to detect the expression of these proteins by Western blot after harvesting these cells in four different ways. B-actin is the housekeeping protein.

The Fibronectin and Laminin expression in cells detached from PCL-PNIPAAm by reduced temperature was higher than the cells detached by trypsinization. However, Collagen 1 was found equally expressing in cells and non-significantly affected by the two different processes. The result was consistent with immunostaining data and indicated in a whole that trypsinization might adversely affect cell structure and physiology by simply degrading some of the structural ECM proteins as well.

**Example 2**

**Materials and Methods**

**Materials**

Polycaprolactone pellets (PCL, Mn 80,000), polyvinyl alcohol (PVA, Mw = 13-23 KDa, 87-89 % hydrolysed) and dichloromethane (DCM) were purchased from Sigma-Aldrich (UK). Deionised water (DI water) used in this study was obtained from an ultrapure water purification system (Elix™, Millipore).

**Preparation of PCL macro-bead**

PCL macro-beads were prepared using an established emulsion method, followed by the evaporation of the solvent used to liquefy the macrosphere polymer.

An aliquot of PCL pellets was dissolved in DCM to obtain 10, 12, 15 and 18 (w/v %) organic phases, while the

PVA was dissolved into DI water to obtain 0.5, 1.0, 1.5, 2.0 and 3.0 (w/v %) inorganic phase.

A needle syringe (containing 5 ml of PCL solution) was placed on a pump which was used to form PCL/DCM solution droplets, which are precursors to the solidified beads. The 5 mL of formed PCL/DCM droplets were collected in a petri dish, containing 10 mL of PVA solution. The PVA solution was then decanted. DCM solvent was allowed to evaporate through the aqueous phase, thus resulting in droplet solidification, over 3 days in a fume hood.

**Results and Discussion**

PCL beads were prepared by the o/w emulsion solvent evaporation process. In the first step, the organic phase was emulsified in the aqueous external phase. The organic phase was dichloromethane. Due to the low evaporation temperature of dichloromethane, the macrospheres formed faster than with other solvents having a higher evaporation temperature, such as chloroform. As the organic solvent evaporates from the surface of the droplets, the concentration of PCL increases and reaches a critical point at which the polymer concentration exceeds its solubility in the organic phase. At this critical point, it precipitates to produce macrospheres. The method used for fabrication of PCL macrospheres is shown in figure 7. The PCL pellets that were prepared from this method are also shown in figure 7 (see "PCL pellets - Oxford") alongside the PCL pellets that were purchased from Sigma-Aldrich ("PCL pellets - Sigma").

***Effect of concentration of PCL on the forming of the beads***

The influence of PCL concentration on the forming of the beads was probed using 10, 12, 15 and 18 wt/v%, as shown in Table 1.

Table 1. Effect of PCL concentration on bead formation

| PCL concentration (wt/v%) | Droplet | Formed the beads (after solidification) | Rate of pump (ml/min) | PVA concentration (wt/v%) |
|---|---|---|---|---|
| 10 | Yes | No | 0.4 | 3 |
| 12 | Yes | No | | |
| 15 | Yes | Yes | | |
| 18 | No | N/A | | |

Droplets were formed at a PCL concentration of 10, 12 and 15 wt/v% while there was no droplet formation at 18 wt/v%. After the solidification step, the beads were obtained from only 15 % wt/v of PCL. Thus, the concentration of PCL at 15 wt/v% was chosen as the optimal concentration for further experiments.

***Effect of flow rates on bead size***

Table 2 shows the relationship between the flow rate and the size of the beads.

Table 2. Effect of flow rates on bead size

| PCL concentration (wt/v%) | PVA concentration (wt/v%) | Rate of pump (ml/min) | Size of beads (mm) |
|---|---|---|---|
| 15 | 3 | 0.2 | < 0.5 |
| | | 0.4 | 0.5 - 2.0 |
| | | 0.8 | 2.0 - 3.0 |
| | | 1.2 | Too fast |

When the flow rate was increased, the size of the beads increased. A higher flow rate at the dispersed phase delivered a larger volume of PCL/DCM solution for each formed droplet. This phenomenon resulted in larger macrobeads formed from PCL/DCM solutions of the same concentration.

***Effect of concentration of PVA on bead size***

PVA was used as the emulsifier. After preparing the macrospheres as described above, the macrospheres were rinsed with DI water several times to remove the PVA.

The hydroxyl groups in PVA interact with the water phase, while the polymer chain interacts with the dichloromethane, which makes the formed emulsion more stable. Variation in PVA concentration and volume can affect the emulsion stability, which can, in turn, affect the size of the macrospheres. As shown in Table 3, an increase in the PVA concentration led to a decrease in the size of the macrospheres.

Table 3. Effect of concentration of PVA on bead size

| PCL concentration (wt/v%) | Rate of pump (ml/min) | PVA concentration (wt/v%) | Size of beads (mm) | Type of beads (depending on the size) |
|---|---|---|---|---|
| 15 | 0.4 | 0.5 | 2.7 - 3.7 | Bead 1 |
| | | 1.0 | 1.8 - 2.2 | Bead 2 |
| | | 1.5 | 1.2 - 1.5 | Bead 3 |
| | | 2.0 | 0.5 - 1.0 | Bead 4 |
| | | 3.0 | 0.5 - 1.0 | Bead 4 |

When the concentration of PVA was increased, more PVA molecules overlay the surface of the droplets, providing increased protection of the droplets against coalescence which resulted in the production of smaller emulsion droplets. Since the macrospheres were formed from emulsion droplets after solvent evaporation, the size was dependent on the size of the emulsion droplets. Furthermore, the viscosity of the aqueous solution was higher at high PVA concentrations compared to lower concentrations, which may be another factor that contributes to the separation of droplets in the emulsion from each other.

**Characterizations and measurements**

Scanning electron microscopy (SEM) and Energy Dispersion Spectroscopy (EDS) analysis: the surface morphology of the PCL and PCL-PNIPAAm macro-carriers was observed by SEM (Carl Zeiss Evo LS15 VP-Scanning Electron Microscope SE, BSE, VPSE, EPSE detectors) at an accelerating voltage of 10 kV. Before the SEM investigation, the samples were coated with gold by sputtering. INCA X-Act X-ray system (Oxford Instruments), OIM XM 4 Hikari EBSD System (EDAX) for EDS analysis.
Fourier transform infrared (FTIR) spectra were recorded using the same equipment and method as described in Example 1.

***Size distribution, morphology and FITR of the PCL macro-beads***

Figure 8 shows the morphology of PCL macro-beads that were prepared using the method described above (see the images labelled (A), which are the "PCL-Oxford" beads) and those purchased from Sigma (see the images labelled (B), which are the "PCL-Sigma" beads). The surface of the bead shown in (A) was porous while the surface of the bead shown in (B) was non-porous and dense. The pores may be formed by the rapid precipitation of PCL, such as by using an organic solvent (e.g. DCM) having a low evaporation temperature during the solidification step. The pores can absorb and retain nutrients and medium on the surface of the beads. The porous surface can support cell adherence and growth better than a dense surface.
As shown in Table 3 above, four types of bead were prepared as described above at 15 wt/v % of PCL, rate of pump at 0.4 ml/min and various concentrations of PVA ranging from 0.5 to 3.0 wt/v%. The beads had diameters ranging from 0.5 to 3.7 mm. Bead 1 had diameters ranging from 2.7-3.7 mm, bead 2 had diameters ranging from 1.8-2.2 mm, bead 3 had diameters ranging from 1.2-1.5 mm, and bead 4 had diameters ranging from 0.5-1.0 mm. The morphology and size distribution of these beads are shown in figure 9. By controlling the PVA concentration and flow rate, the size of the beads could be controlled to obtain a uniform size. The average size of bead 1 was 3.09 mm, bead 2 was 1.89 mm, bead 3 was 1.37 mm and bead 4 was 0.83 mm.
Results of the FITR spectra of PCL-Oxford and PCL-Sigma performed are shown in figure 10. These results showed that all the PCL-Oxford peaks matched all the PCL-Sigma peaks, which confirmed that the fabrication process did not change the chemical structure of PCL.

***Morphology of PCL and PCL-PNIPAAm macro-beads***

The porous PCL macro-beads (e.g. "PCL-Oxford" beads) were coated with PNIPAAm using the method described in Example 1. The surface morphology of the PCL and the resulting PCL-PNIPAAm macro-beads was characterized by SEM as shown in figure 11 (the PCL-PNIPAAm macro-beads are labelled "PCL-P" in the figure). The step of grafting PNIPAAm onto the porous PCL beads did not affect their surface morphology. This can be seen from figure 12,

which shows a pore on the surface of a PCL-PNIPAAm macro-bead.

The appearance of a nitrogen peak in the EDS images (see figure 11) for the PCL-PNIPAAm (see between the C and O peaks in the EDS image for "PCL-P) confirmed that the polymerization had been carried out properly. The surface morphology of the commercially available PCL beads (e.g. "PCL Sigma") was unaffected by the presence of the PNIPAAm coating.

**Cell culture on macrocarriers**

Green Fluorescence Protein (GFP) was cloned into Mesenchymal stem cells (MSC, provided by the Department of Paediatrics and Adolescent Medicine, LKS Faculty of Medicine, The University of Hong Kong). Briefly, primary mesenchymal cells (obtained from unfractionated bone marrow mononuclear cells of a healthy donor) were cultured for 2 months. Cells were infected with a VSV-G (expressing the G glycoprotein of the vesicular stomatitis virus) pseudotyped retroviral vector that contained the hTERT and green fluorescent protein (GFP) genes, separated by an internal ribosome entry site (IRES), under the control of the murine stem cell virus (MSCV) long-terminal repeat (LTR). The GFP+ and GFP-MSC then were separated with a fluorescence-activated cell sorter (MoFlo, Cytomation, Fort Collins, CO, USA) 6. MSC-GFP were cultures in Dulbecco's modified Eagle's medium (DMEM 1.0 mg/l of glucose, Gibco BRL, Gaithersburg, MD, USA) supplemented with 10% (v/v) fetal bovine serum (FBS, Gibco BRL) and 0.1% (v/v) penicillin-streptomycin (PS, Gibco BRL).

The PCL and PCL-PNIPAAm macro-beads prepared above were placed in a laminar hood and UV radiation was applied for 30 minutes. Next, the PCL and PCL-PNIPAAm beads were immersed in 70% ethanol for 3 hours, washed with phosphate buffer saline (PBS) for 10 min and incubated in DMEM at 37 °C, overnight. Cell seeding density was $2.8 \times 10^5$ cells/ml.

Cell proliferation of MSC seeded onto PCL and PCL-PNIPAAm after 3 and 7 days of incubation as determined by Hoechst staining and Green fluorescence protein (GFP) images are shown in figures 13 and 14. Figure 13 shows the cell proliferation of MSC seeded on PCL (see (A)) and PCL-PNIPAAm (see (B)) after 3 and 7 days, stained by Hoechst (Nuclei staining in blue dot), observed by fluorescence microscope in low magnification. Figure 14 shows the cell proliferation of MSC seeded on PCL (see (A)) and PCL-PNIPAAm (see (B)) after 3 and 7 days, stained by Hoechst (Nuclei staining in blue dot) and green fluorescence protein, observed my fluorescence microscope in high magnification. The black background including the large blue dot areas are the beads.

The number of cells (the blue dot) on both PCL and PCL-PNIPAAm increased with increasing culture time from 3 days to 7 days. PCL used in this work had a molecular weight of 80 kDa (PCL80k). Very dense and clustered cells with higher viability were observed at day 7 and on grafted surfaces (PCL-PNIPAAm) than at day 3 and non-grafted PCL surfaces. Notably both groups of cells were healthy and showed their normal physiology and spindle shaped appearance. These results indicated that PNIPAAm grafting onto PCL surfaces was risk-free and can provide a valuable tool for recovering large-scale cellular collections.

***Cell proliferation***

*First study*

For cell viability and proliferation, the CCK-8 assay (Sigma) was performed after 1, 7, 14 and 21 days of incubation. Cell seeding density was $5 \times 10^3$ cells/ml. For this experiment, around 20 g of PCL beads were prepared in the laboratory.

In this time dependent study, MSC proliferation was shown from 1 to 21 days on PCL-PNIPAAm and growth-proliferation was compared to different controls, which included on tissue culture plate (TCP), non-coated PCL beads and PCL beads from Sigma. The results are shown in figure 15.

At day 1, cells were found to be growing non-significantly on all sorts of surfaces. At day 7, cells proliferated significantly higher in TCP controls than with the PNIPAAm coated PCL surfaces and the commercially available PCL-beads ("PCL-Sigma"). There was no significant difference in proliferation between the cells on the non-coated PCL and the cells on the PCL-PNIPAAm samples. However, there was a slight significant difference in cell proliferation between (a) the non-coated PCL surfaces and the commercially available PCL-beads ($p<0.01$) and (b) the PCL-PNIPAAm samples and the commercially available PCL-beads ($p<0.05$).

At day 14, cells were found to be proliferating on all samples and controls. Proliferated cell numbers on non-coated PCL, PCL-PNIPAAm and the commercially available PCL-beads were measured to be non-significant. Cell proliferation on TCP was significantly lower than any other surface at day 14. On day 21, cell growth on non-coated PCL, PCL-PNIPAAm and PCL-Sigma was significantly high compared to cell proliferation on TCP.

Over the time-scale, an exponential proliferation of MSCs on all surfaces can be seen. At day 21, as compared to days 14 and 21, the cells were found to be in a stationary phase and a non-exponential growth pattern was observed.

This is simply due to confluent growth of MSCs; there remains no room for any new cell growth in the culture.

*Second study*

In this second study, the same procedure was used as in the first study except that the cell seeding density was $1 \times 10^4$ cells/ml and the CCK-8 assay (Sigma) was performed after 1, 3 and 7 days of incubation. The results are shown in figure 16.

At day 3, the cells grown on the porous PCL and the porous PCL-PNIPAAm surfaces exhibited similar behaviour. However, a significant ($p < 0.0001$) increase in OD was observed with porous PCL compared to the commercially available PCL-beads available PCL-beads ("PCL-Sigma"). The cell viability at day 7 had the same pattern as day 3, except that more cells survived on the surfaces of porous PCL beads than on the surfaces of the commercially available beads. No significant difference in cell viability for PCL and PCL-PNIPAAm surfaces was observed.

From the results, it can be seen that the cells grew better on the porous PCL beads than on the commercially available PCL-beads.

***Cell detachment from PCL-PNIPAAm***

MSC detachment from PCL-PNIPAAm surfaces was observed under fluorescence microscopy. GFP loaded MSCs were grown on the sample surfaces overnight at 37 °C. After this initial attachment, the incubation temperature was reduced from 37 °C to 25 °C for at least 1 hour. The detached cells from PCL-PNIPAAm were observed and imaged by an inverted microscope (Eclipse Ti, Nilon). Green fluorescence from the cells indicated free cells in the culture. Figure 17 shows the MSCs detached from PCL-PNIPAAm at (a) low magnification and (b) high magnification.

## Claims

1. A macrocarrier for the propagation of biological cells, said macrocarrier comprising substrate particles that are coated with a thermoresponsive polymer that provides the macrocarrier with a cell-receiving surface and responding to a change in temperature to release cells from the macrocarrier, wherein:

    the cell-receiving surface is porous;
    each substrate particle has a spherical, ellipsoid, ovoid or ring shape; and
    at least 50% of the substrate particles have a particle size of at least 1 mm, wherein the particle size is the largest linear dimension across the substrate particle.

2. A macrocarrier as claimed in claim 1, wherein at least 90% of the substrate particles have a particle size of 2 to 6 mm.

3. A macrocarrier as claimed in claim 1 or claim 2, wherein the substrate particles are partially coated with the thermoresponsive polymer.

4. A macrocarrier as claimed in any one of the preceding claims, wherein the substrate particle is formed of a biocompatible material, wherein the material is biocompatible according to ISO 10993 and optionally is selected from polysaccharide, protein, glass, polystyrene, polyester, polyolefin, silica, silicone, polyacrylamide and polyacrylate.

5. A macrocarrier as claimed in any one of the preceding claims, wherein the substrate particle comprises polycaprolactone.

6. A macrocarrier as claimed in any one of the preceding claims, wherein the thermo-responsive polymer provides the macrocarrier with a cell-receiving surface above a threshold temperature and releases cells from the macrocarrier below the threshold temperature.

7. A macrocarrier as claimed in claim 6, wherein the threshold temperature is a temperature of 20 to 40 degrees C.

8. A macrocarrier as claimed in claim 1, wherein the thermoresponsive polymer is capable of transforming from a hydrophilic, cell-receiving state to a hydrophobic, cell-releasing state in response to a change in temperature.

9. A macrocarrier as claimed in any one of the preceding claims, wherein the thermoresponsive polymer comprises poly(N-isopropyl acrylamide) (PNIPAAm).

**10.** A macrocarrier as claimed in claim 6, wherein the substrate particles comprise polycaprolactone and wherein the poly(N-isopropyl acrylamide) (PNIPAAm) is coupled to the polycaprolactone via an amide linkage.

**11.** A macrocarrier as claimed in any one of the preceding claims, which further comprises biological cells attached to the thermoresponsive polymer, optionally wherein the biological cells are selected from mesenchymal stem cells ("MSCs"), human dermal fibroblasts ("HDFs"), human umbilical vein endothelial cells (HUVEC) and neuroblastoma Sy5y cells.

**12.** A system for the propagation of biological cells, said system comprising a bio-reactor and a macrocarrier as claimed in any one of claims 1 to 11.

**13.** A process for the propagation of biological cells, said process comprising:

a. contacting biological cells with a macrocarrier as claimed in any one of claims 1 to 11 in a cell culturing medium;
b. propagating the cells on the macrocarrier by subjecting the macrocarrier to a temperature at which the thermoresponsive polymer presents a cell-receiving surface; and
c. altering the temperature of the macrocarrier to release any propagated cells from the macrocarrier.

**14.** A process as claimed in claim 13, wherein the biological cells are contacted with the macrocarrier in a bioreactor.

**15.** A process as claimed in claim 13 or 14, wherein the macrocarrier is exposed to a first temperature of 20 to 40 degrees to propagate the cells, and wherein the temperature of the macrocarrier is reduced below the first temperature to release the propagated cells.

## Patentansprüche

**1.** Makroträger zur Vermehrung biologischer Zellen, wobei der Makroträger Substratpartikel umfasst, die mit einem thermoresponsiven Polymer beschichtet sind, das dem Makroträger eine zellaufnehmende Oberfläche bereitstellt und auf eine Temperaturänderung reagiert, um Zellen von dem Makroträger freizusetzen, wobei:

die zellaufnehmende Oberfläche porös ist;
jedes Substratpartikel eine Kugel-, Ellipsoid-, Ovoid- oder Ringform aufweist; und
mindestens 50 % der Substratpartikel eine Partikelgröße von mindestens 1 mm aufweisen, wobei die Partikelgröße die größte lineare Abmessung über das Substratpartikel ist.

**2.** Makroträger nach Anspruch 1, wobei mindestens 90 % der Substratpartikel eine Partikelgröße von 2 bis 6 mm aufweisen.

**3.** Makroträger nach Anspruch 1 oder Anspruch 2, wobei die Substratpartikel teilweise mit dem thermoresponsiven Polymer beschichtet sind.

**4.** Makroträger nach einem der vorhergehenden Ansprüche, wobei das Substratpartikel aus einem biokompatiblen Material gebildet ist, wobei das Material gemäß ISO 10993 biokompatibel ist und optional aus Polysaccharid, Protein, Glas, Polystyrol, Polyester, Polyolefin, Siliciumdioxid, Silicon, Polyacrylamid und Polyacrylat ausgewählt ist.

**5.** Makroträger nach einem der vorhergehenden Ansprüche, wobei das Substratpartikel Polycaprolacton umfasst.

**6.** Makroträger nach einem der vorhergehenden Ansprüche, wobei das thermoresponsive Polymer über einer Schwellentemperatur dem Makroträger eine zellaufnehmende Oberfläche bereitstellt und unter der Schwellentemperatur Zellen von dem Makroträger freisetzt.

**7.** Makroträger nach Anspruch 6, wobei die Schwellentemperatur eine Temperatur von 20 bis 40 Grad C ist.

**8.** Makroträger nach Anspruch 1, wobei das thermoresponsive Polymer dazu in der Lage ist, sich als Reaktion auf eine Temperaturänderung von einem hydrophilen, zellaufnehmenden Zustand in einen hydrophoben, zellfreisetzenden Zustand umzuwandeln.

**9.** Makroträger nach einem der vorhergehenden Ansprüche, wobei das thermoresponsive Polymer Poly(N-isopropylacrylamid) (PNIPAAm) umfasst.

**10.** Makroträger nach Anspruch 6, wobei die Substratpartikel Polycaprolacton umfassen und wobei das Poly(N-isopropylacrylamid) (PNIPAAm) über eine Amidbindung an das Polycaprolacton gekoppelt ist.

**11.** Makroträger nach einem der vorhergehenden Ansprüche, der ferner biologische Zellen umfasst, die an dem thermoresponsiven Polymer angebracht sind, wobei optional die biologischen Zellen aus mesenchymalen Stammzellen ("MSCs"), humanen dermalen Fibroblasten ("HDFs"), Endothelzellen aus humanen Nabelschnurvenen (HUVEC) und Neuroblastom-Sy5y-Zellen ausgewählt sind.

**12.** System zur Vermehrung biologischer Zellen, wobei das System einen Bioreaktor und einen Makroträger nach einem der Ansprüche 1 bis 11 umfasst.

**13.** Vorgang zur Vermehrung biologischer Zellen, wobei der Vorgang Folgendes umfasst:

a. Kontaktieren biologischer Zellen mit einem Makroträger nach einem der Ansprüche 1 bis 11 in einem Zellkulturmedium;
b. Vermehren der Zellen auf dem Makroträger, indem der Makroträger einer Temperatur unterworfen wird, bei der das thermoresponsive Polymer eine zellaufnehmende Oberfläche darstellt; und
c. Verändern der Temperatur des Makroträgers, um etwaige vermehrte Zellen von dem Makroträger freizusetzen.

**14.** Vorgang nach Anspruch 13, wobei die biologischen Zellen in einem Bioreaktor mit dem Makroträger kontaktiert werden.

**15.** Vorgang nach Anspruch 13 oder 14, wobei der Makroträger einer ersten Temperatur von 20 bis 40 Grad ausgesetzt wird, um die Zellen zu vermehren, und wobei die Temperatur des Makroträgers unter die erste Temperatur reduziert wird, um die vermehrten Zellen freizusetzen.

## Revendications

**1.** Macrosupport destiné à la propagation de cellules biologiques, ledit macrosupport comprenant des particules de substrat qui sont enrobées d’un polymère thermosensible qui fournit au macrosupport une surface de réception de cellules et réagissant à un changement de température de manière à libérer des cellules du macrosupport :

ladite surface de réception de cellules étant poreuse ;
chaque particule de substrat comportant une forme sphérique, ellipsoïde, ovoïde ou annulaire ; et
au moins 50 % des particules de substrat comportant une taille de particule d’au moins 1 mm, ladite taille de particule étant la dimension linéaire la plus grande à travers la particule de substrat.

**2.** Macrosupport selon la revendication 1, au moins 90 % des particules de substrat comportant une taille de particule de 2 à 6 mm.

**3.** Macrosupport selon la revendication 1 ou la revendication 2, lesdits particules de substrat étant partiellement enrobées avec le polymère thermosensible.

**4.** Macrosupport selon l’une quelconque des revendications précédentes, ladite particule de substrat étant formée d’un matériau biocompatible, ledit matériau étant biocompatible selon la norme ISO 10993 et étant éventuellement choisi parmi un polysaccharide, une protéine, le verre, le polystyrène, un polyester, une polyoléfine, la silice, un silicone, un polyacrylamide et un polyacrylate.

**5.** Macrosupport selon l’une quelconque des revendications précédentes, ladite particule de substrat comprenant de la polycaprolactone.

**6.** Macrosupport selon l’une quelconque des revendications précédentes, ledit polymère thermosensible fournissant au macrosupport une surface de réception de cellules au-dessus d’une température seuil et libérant des cellules

du macrosupport en dessous de la température seuil.

**7.** Macrosupport selon la revendication 6, ladite température seuil étant une température de 20 à 40 degrés C.

**8.** Macrosupport selon la revendication 1, ledit polymère thermosensible étant en mesure de passer d'un état de réception de cellules hydrophile à un état de libération de cellules hydrophobe en réponse à un changement de température.

**9.** Macrosupport selon l'une quelconque des revendications précédentes, ledit polymère thermosensible comprenant du polyacrylamide de N-isopropyle (PNIPAAm).

**10.** Macrosupport selon la revendication 6, lesdites particules de substrat comprenant de la polycaprolactone et ledit polyacrylamide de N-isopropyle (PNIPAAm) étant couplé à la polycaprolactone par l'intermédiaire d'une liaison amide.

**11.** Macrosupport selon l'une quelconque des revendications précédentes, qui comprend en outre des cellules biologiques fixées au polymère thermosensible, éventuellement lesdites cellules biologiques étant choisies parmi les cellules souches mésenchymateuses (« MSC »), les fibroblastes dermiques humains (« HDF »), les cellules endothéliales de veine ombilicale humaines (HUVEC) et les cellules Sy5y de neuroblastome.

**12.** Système destiné à la propagation de cellules biologiques, ledit système comprenant un bioréacteur et un macrosupport selon l'une quelconque des revendications 1 à 11.

**13.** Procédé de propagation de cellules biologiques, ledit procédé comprenant :

a. la mise en contact de cellules biologiques avec un macrosupport selon l'une quelconque des revendications 1 à 11 dans un milieu de culture cellulaire ;
b. la propagation des cellules sur le macrosupport en soumettant le macrosupport à une température à laquelle le polymère thermosensible présente une surface de réception de cellules ; et
c. la modification de la température du macrosupport pour libérer des cellules propagées du macrosupport.

**14.** Procédé selon la revendication 13, lesdites cellules biologiques étant mises en contact avec le macrosupport dans un bioréacteur.

**15.** Procédé selon la revendication 13 ou 14, ledit macrosupport étant exposé à une première température de 20 à 40 degrés de manière à propager les cellules, et ladite température du macrosupport étant réduite en dessous de la première température de manière à libérer les cellules propagées.

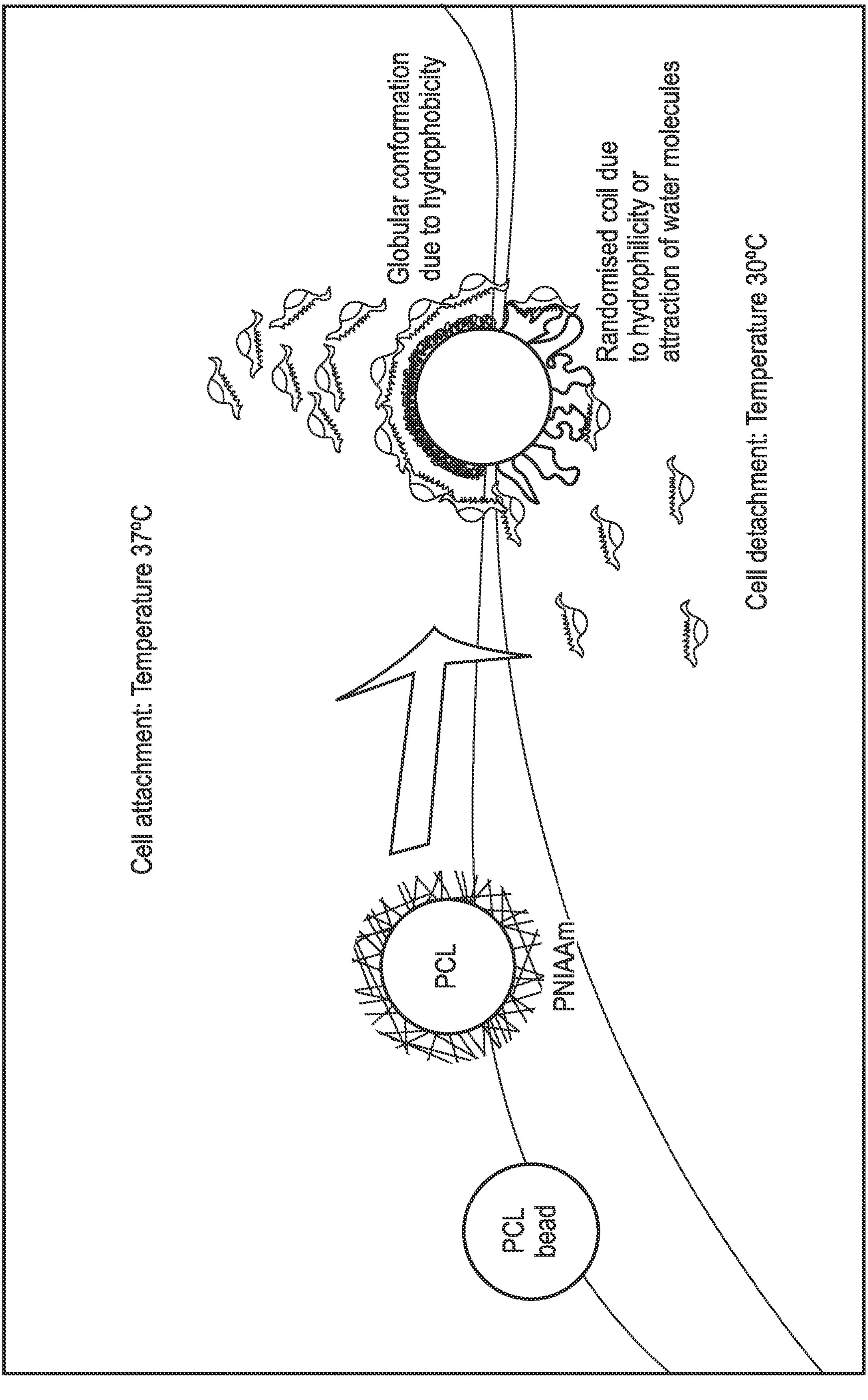

Cell attachment: Temperature 37°C
Globular conformation due to hydrophobicity
Randomised coil due to hydrophilicity or attraction of water molecules
Cell detachment: Temperature 30°C
PCL
PNIAAm
PCL bead


FIG. 1

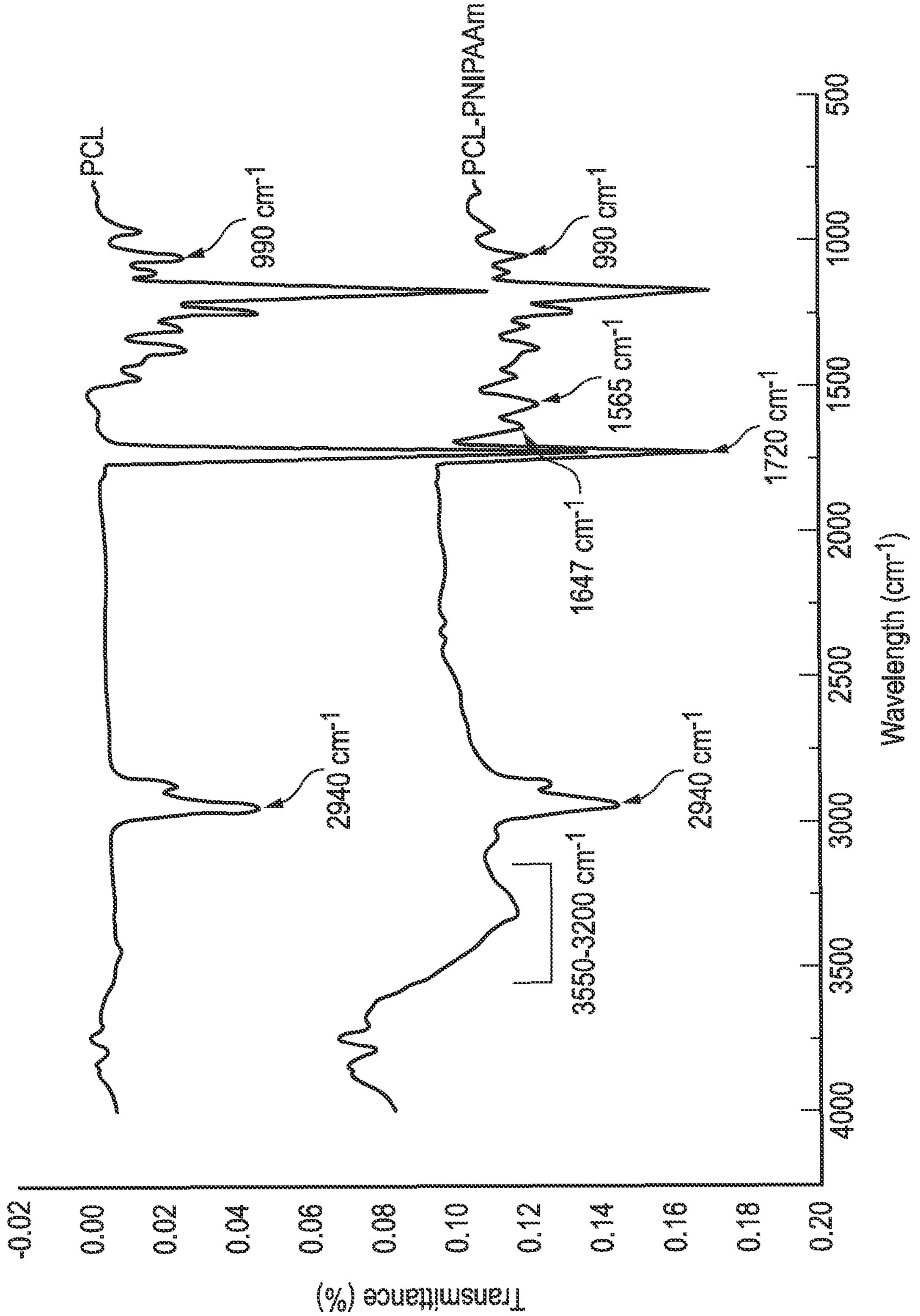

PCL
PCL-PNIPAAm
990 cm-1
990 cm-1
1565 cm-1
1720 cm-1
1647 cm-1
2940 cm-1
2940 cm-1
3550-3200 cm-1
Transmittance (%)
Wavelength (cm-1)
-0.02
0.00
0.02
0.04
0.06
0.08
0.10
0.12
0.14
0.16
0.18
0.20
4000
3500
3000
2500
2000
1500
1000
500


FIG. 2(a)

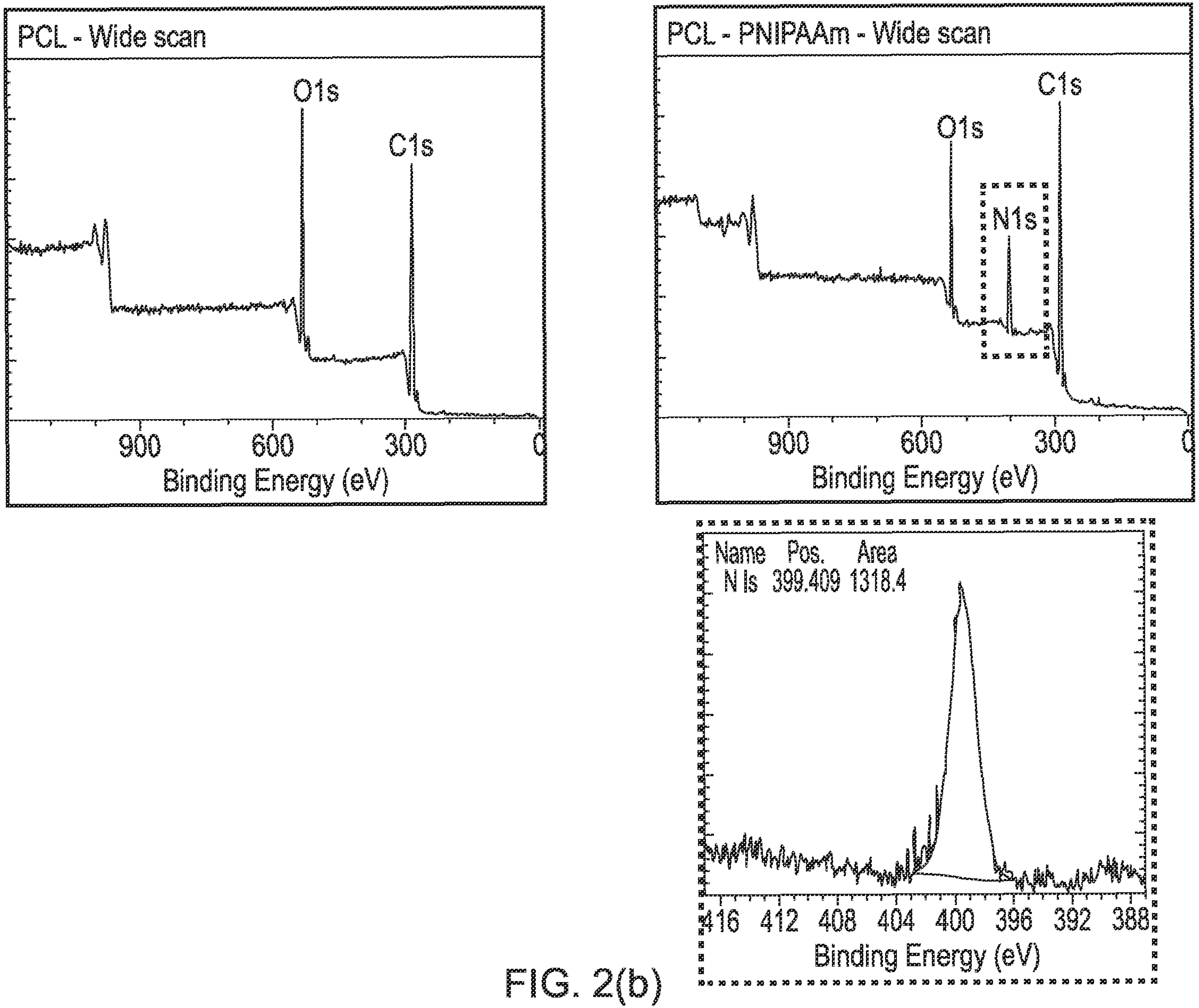
PCL - Wide scan
O1s
C1s
900
600
300
0
Binding Energy (eV)
PCL - PNIPAAm - Wide scan
O1s
C1s
N1s
900
600
300
0
Binding Energy (eV)
Name Pos. Area
N ls 399.409 1318.4
416 412 408 404 400 396 392 388
Binding Energy (eV)

FIG. 2(b)

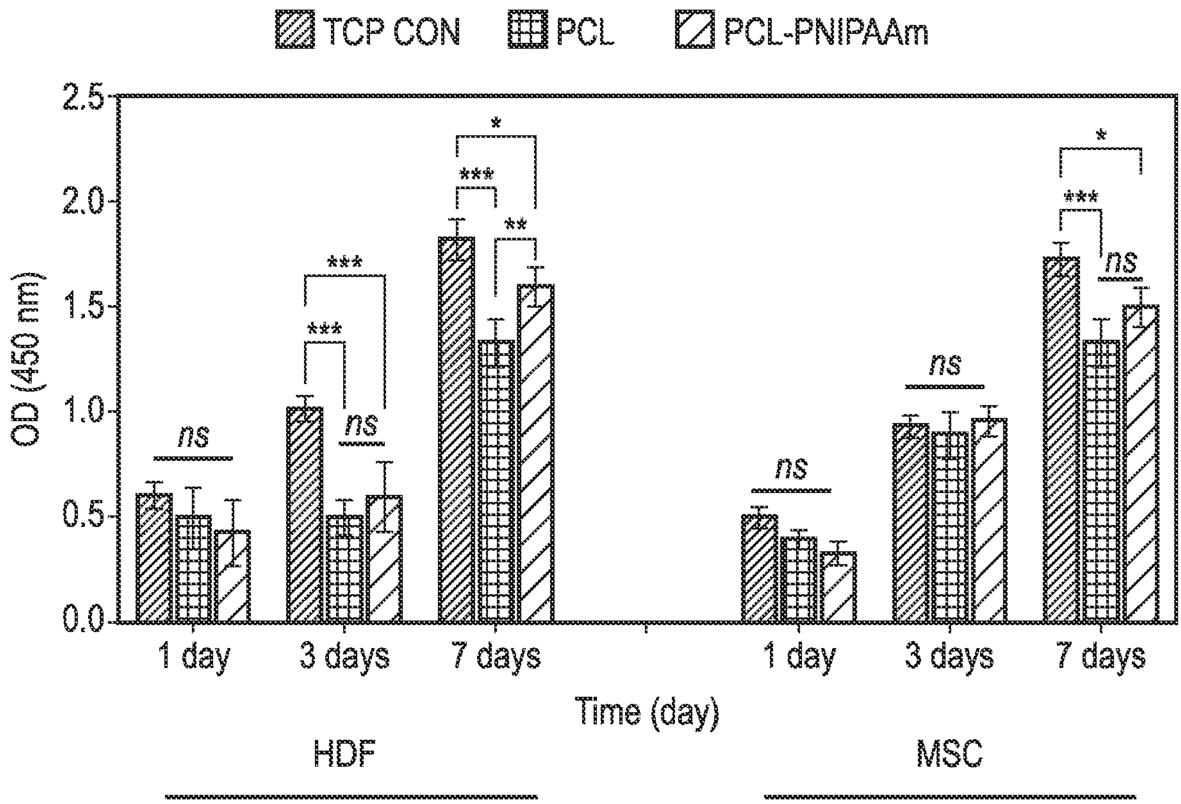
TCP CON
PCL
PCL-PNIPAAm
OD (450 nm)
2.5
2.0
1.5
1.0
0.5
0.0
ns
***
***
ns
*
***
**
ns
ns
*
***
ns
1 day
3 days
7 days
1 day
3 days
7 days
Time (day)
HDF
MSC

FIG. 3

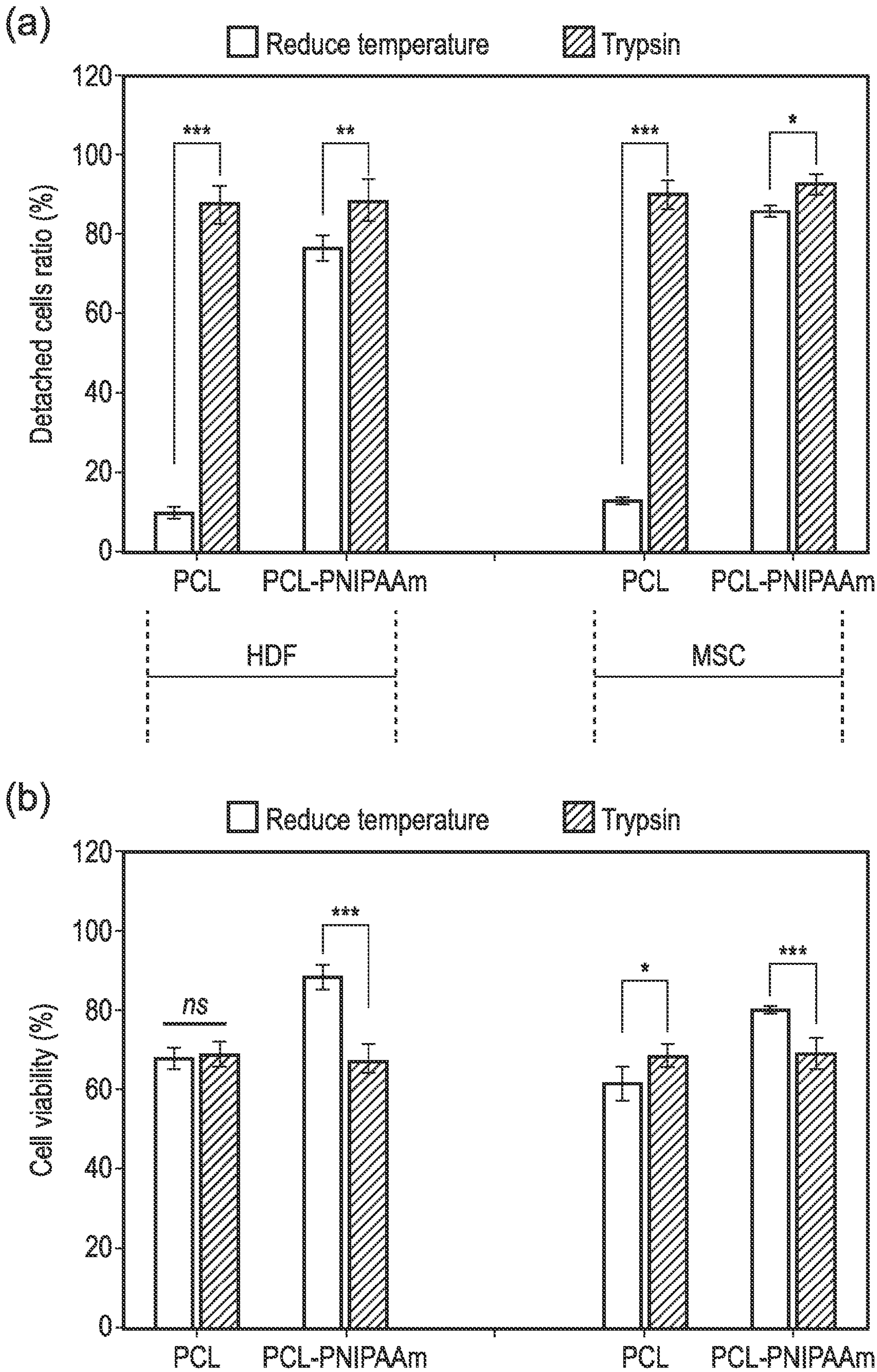
(a)
Reduce temperature
Trypsin
Detached cells ratio (%)
120
100
80
60
40
20
0
***
**
***
*
PCL
PCL-PNIPAAm
PCL
PCL-PNIPAAm
HDF
MSC
(b)
Reduce temperature
Trypsin
Cell viability (%)
120
100
80
60
40
20
0
ns
***
*
***
PCL
PCL-PNIPAAm
PCL
PCL-PNIPAAm

FIG. 4

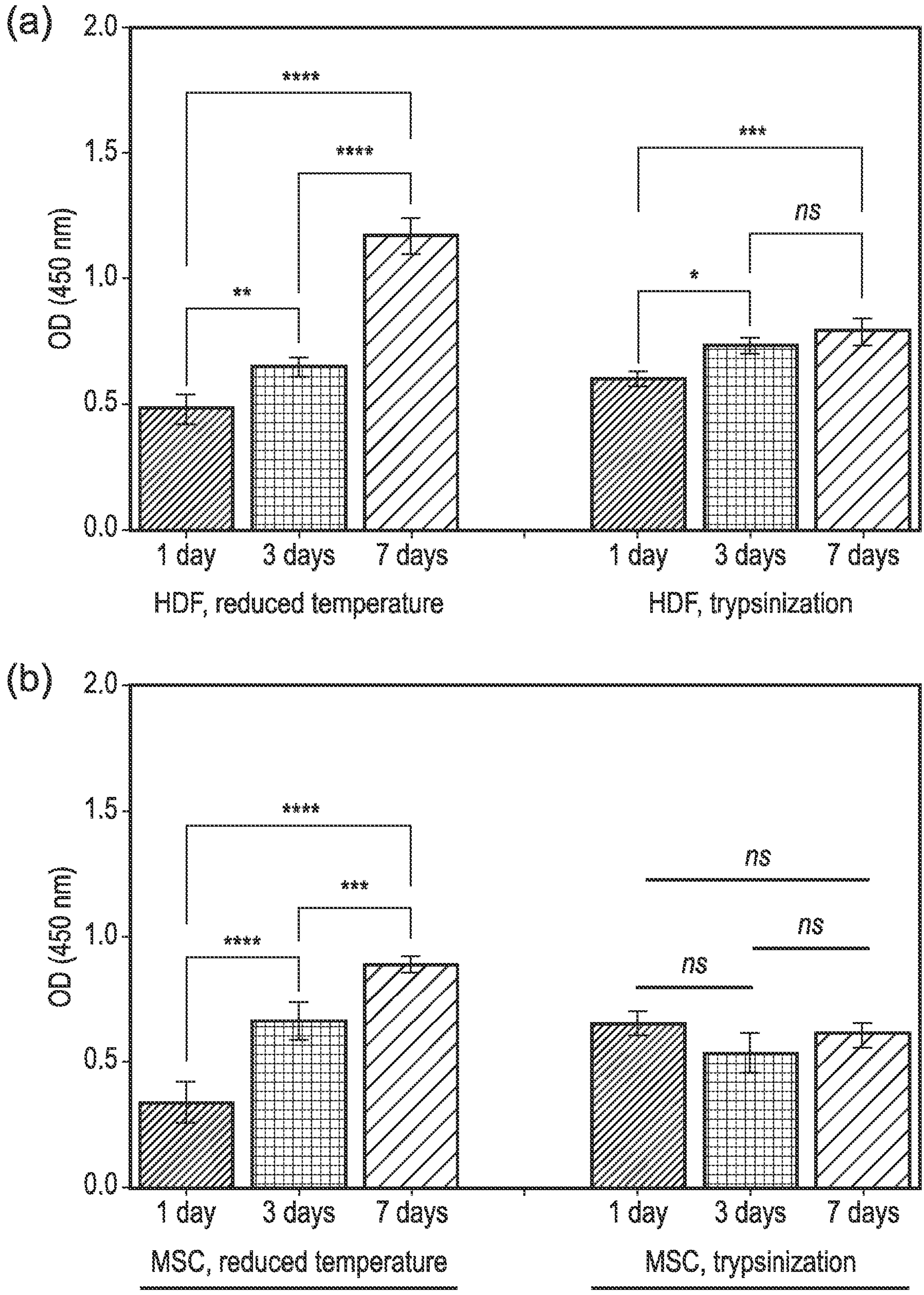
(a)
OD (450 nm)
2.0
1.5
1.0
0.5
0.0
****
****
**
***
ns
*
1 day
3 days
7 days
1 day
3 days
7 days
HDF, reduced temperature
HDF, trypsinization
(b)
OD (450 nm)
2.0
1.5
1.0
0.5
0.0
****
***
****
ns
ns
ns
1 day
3 days
7 days
1 day
3 days
7 days
MSC, reduced temperature
MSC, trypsinization

FIG. 5

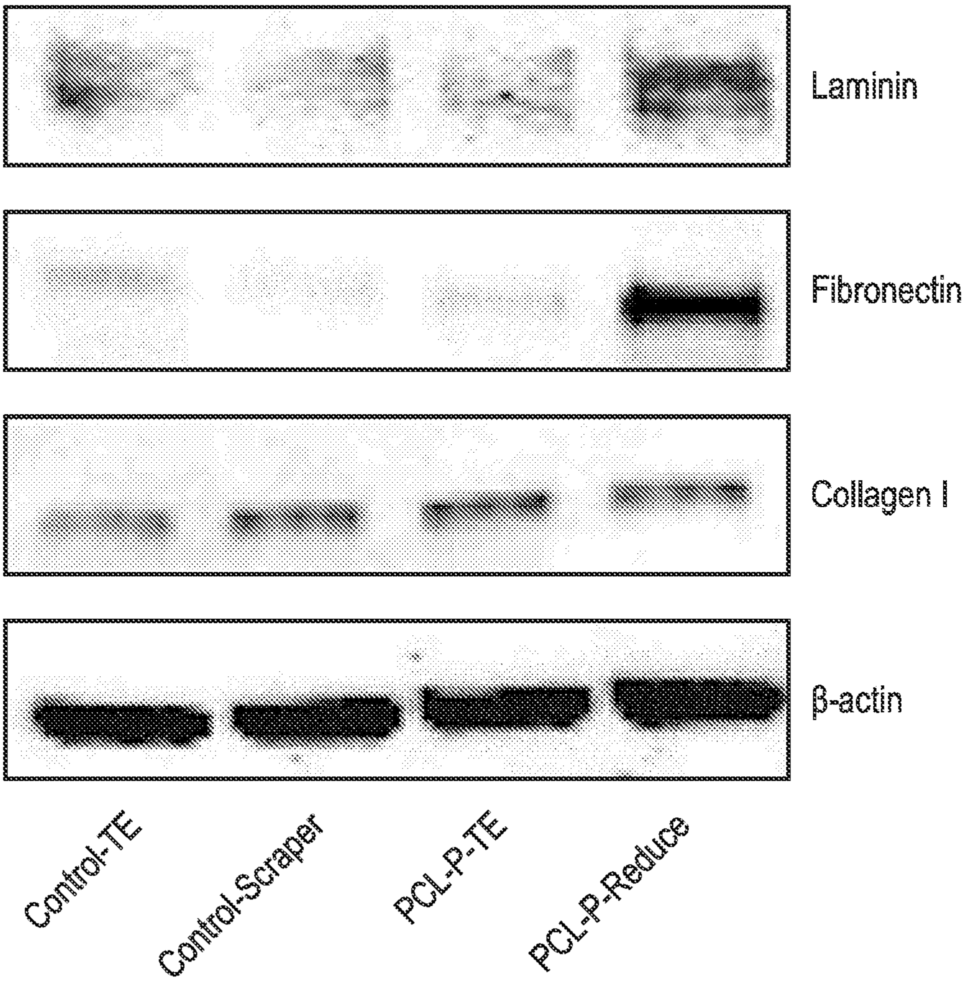
Laminin
Fibronectin
Collagen I
β-actin
Control-TE
Control-Scraper
PCL-P-TE
PCL-P-Reduce

FIG. 6

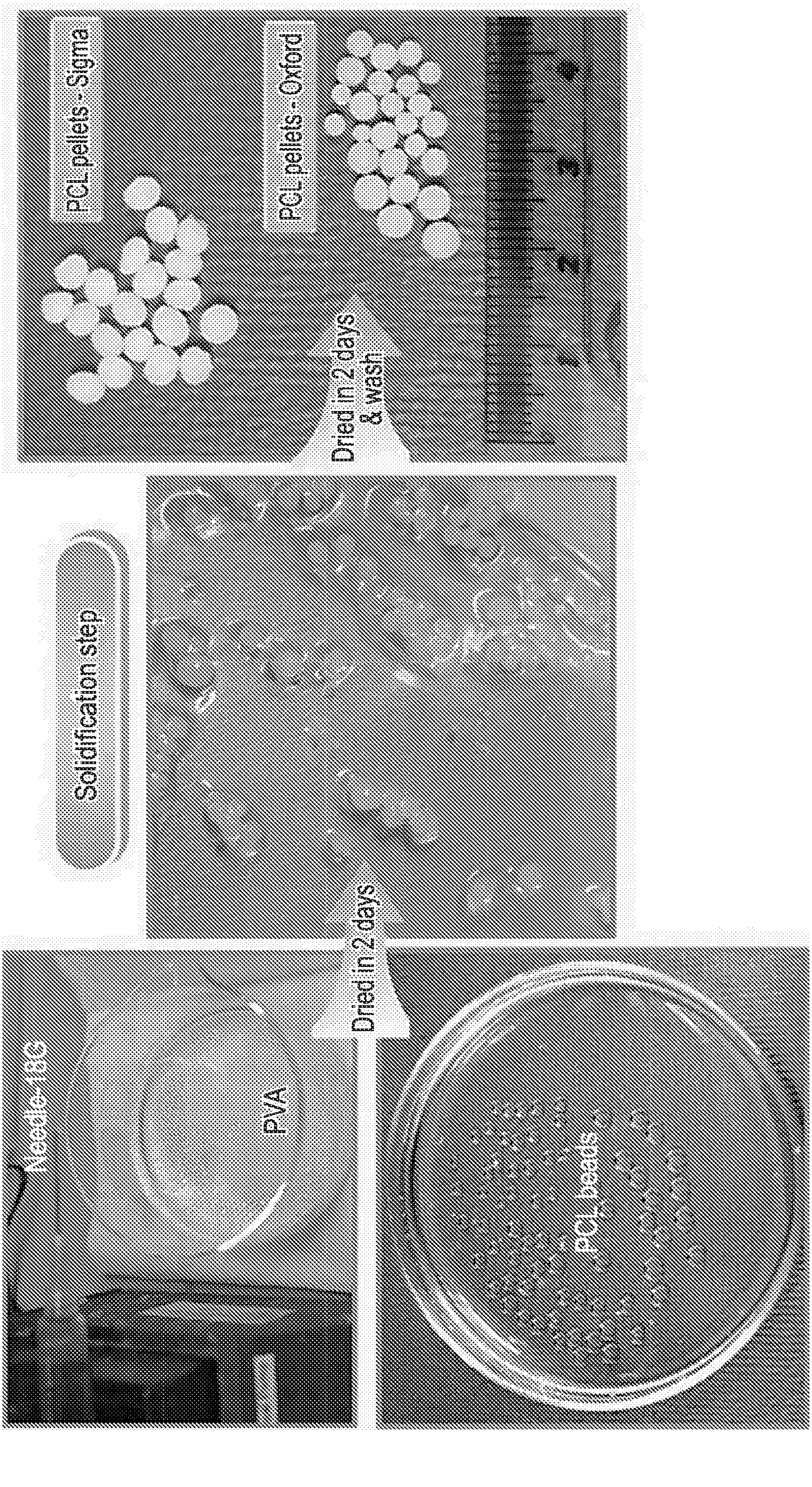
Needle-18G
PVA
Dried in 2 days
PCL beads
Solidification step
Dried in 2 days & wash
PCL pellets - Sigma
PCL pellets - Oxford
1
2
3
4

FIG. 7

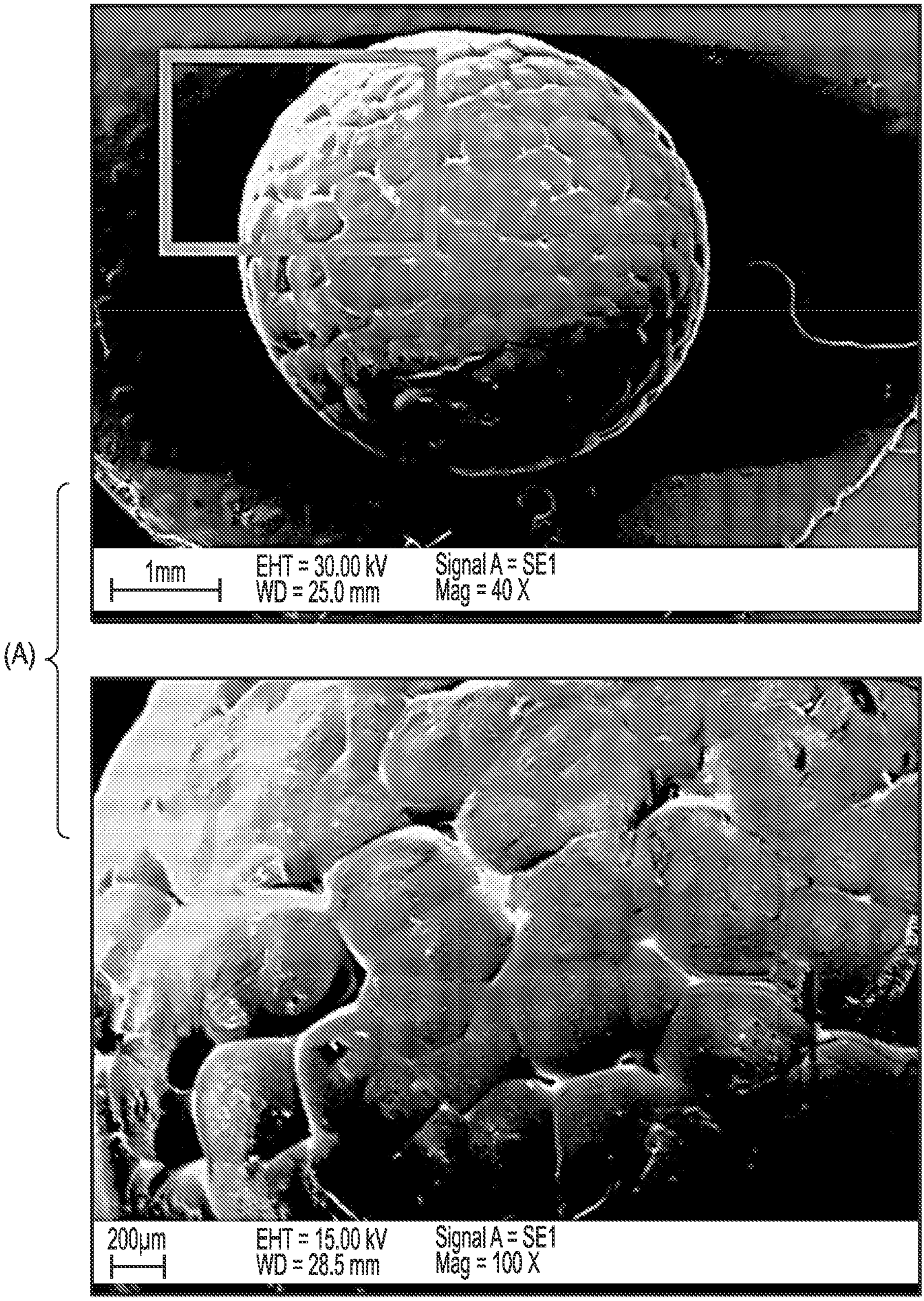
(A)
1mm
EHT = 30.00 kV
WD = 25.0 mm
Signal A = SE1
Mag = 40 X
200µm
EHT = 15.00 kV
WD = 28.5 mm
Signal A = SE1
Mag = 100 X

FIG. 8

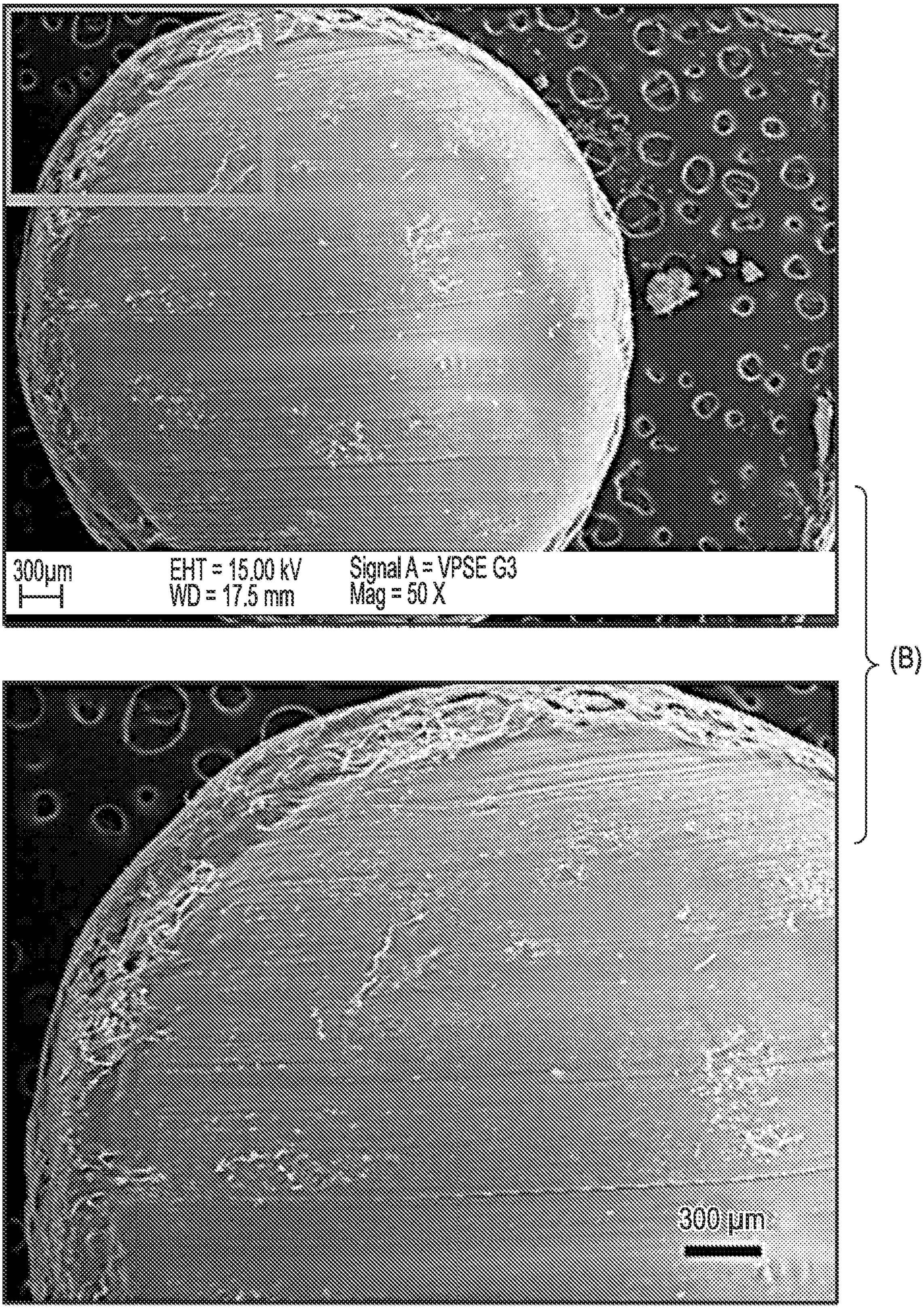


FIG. 8 (Continued)

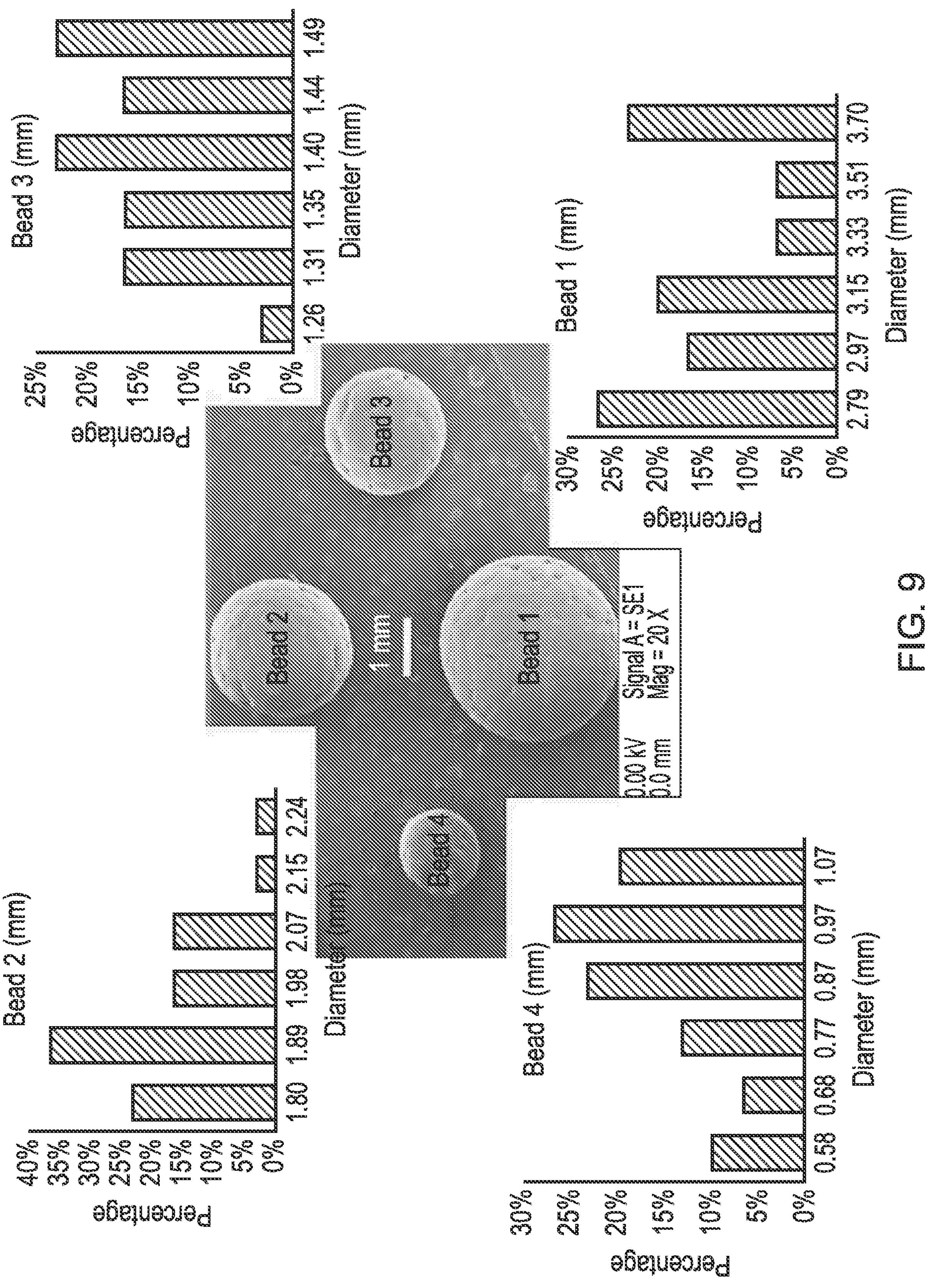

Bead 3 (mm)
Percentage
25%
20%
15%
10%
5%
0%
1.26
1.31
1.35
1.40
1.44
1.49
Diameter (mm)
Bead 1 (mm)
Percentage
30%
25%
20%
15%
10%
5%
0%
2.79
2.97
3.15
3.33
3.51
3.70
Diameter (mm)
Bead 2 (mm)
Percentage
40%
35%
30%
25%
20%
15%
10%
5%
0%
1.80
1.89
1.98
2.07
2.15
2.24
Diameter (mm)
Bead 4 (mm)
Percentage
30%
25%
20%
15%
10%
5%
0%
0.58
0.68
0.77
0.87
0.97
1.07
Diameter (mm)
Bead 1
Bead 2
Bead 3
Bead 4
1 mm
0.00 kV
0.0 mm
Signal A = SE1
Mag = 20 X


FIG. 9

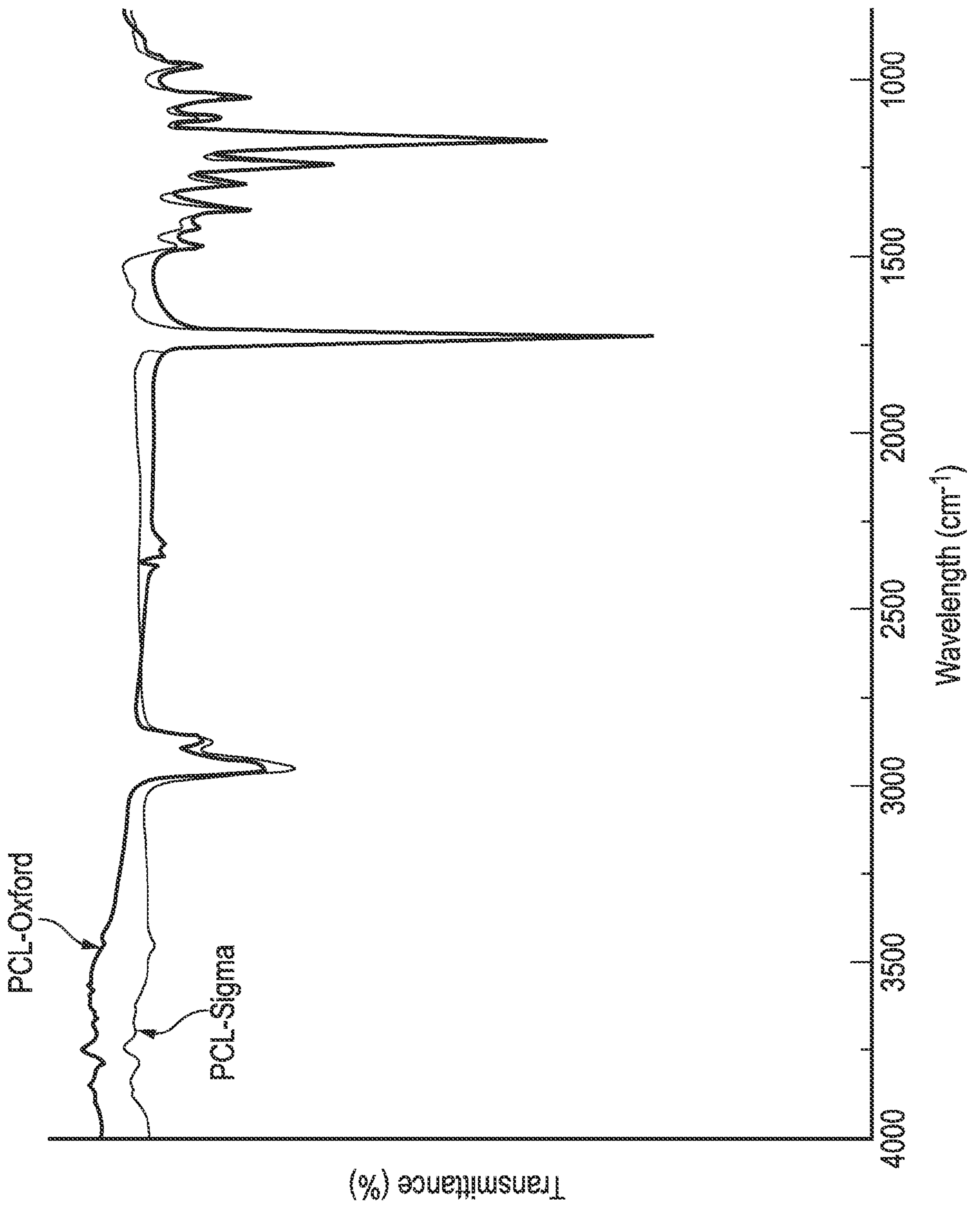
PCL-Oxford
PCL-Sigma
Transmittance (%)
4000
3500
3000
2500
2000
1500
1000
Wavelength (cm-1)

FIG. 10

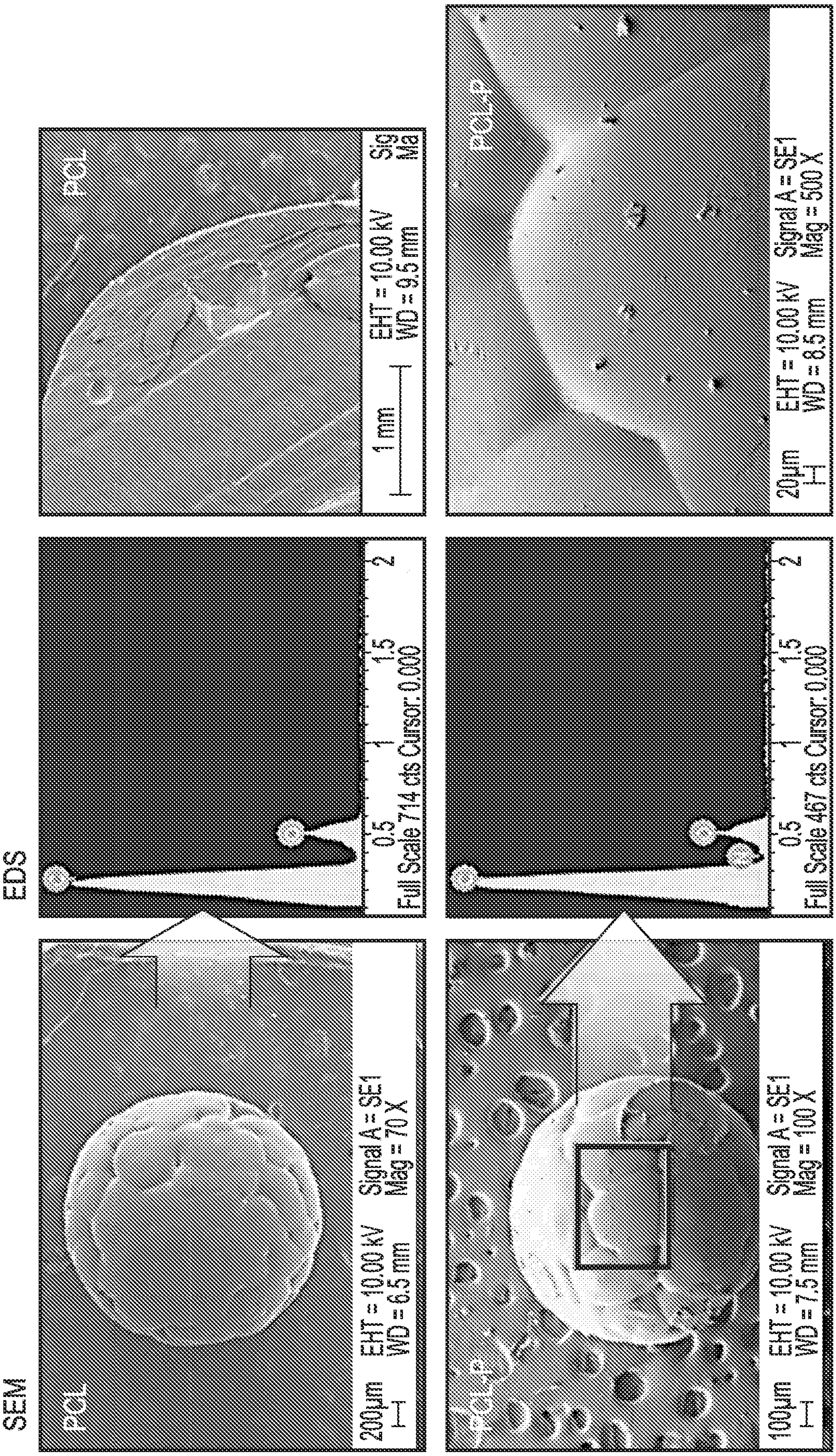
SEM
EDS
PCL
200µm
EHT = 10.00 kV
WD = 6.5 mm
Signal A = SE1
Mag = 70 X
0.5
1
1.5
2
Full Scale 714 cts Cursor: 0.000
PCL
1 mm
EHT = 10.00 kV
WD = 9.5 mm
PCL-P
100µm
EHT = 10.00 kV
WD = 7.5 mm
Signal A = SE1
Mag = 100 X
Full Scale 467 cts Cursor: 0.000
PCL-P
20µm
EHT = 10.00 kV
WD = 8.5 mm
Signal A = SE1
Mag = 500 X

FIG. 11

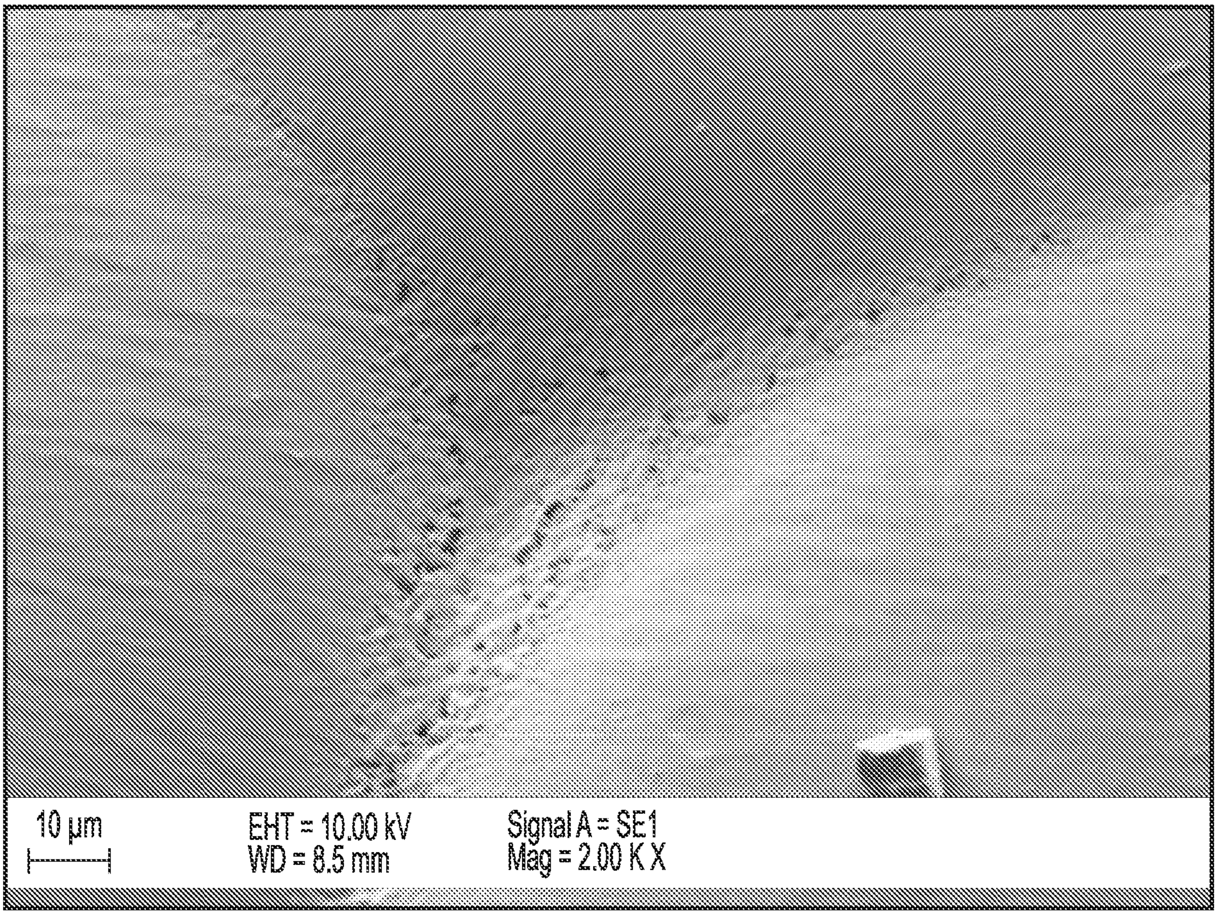


FIG. 12

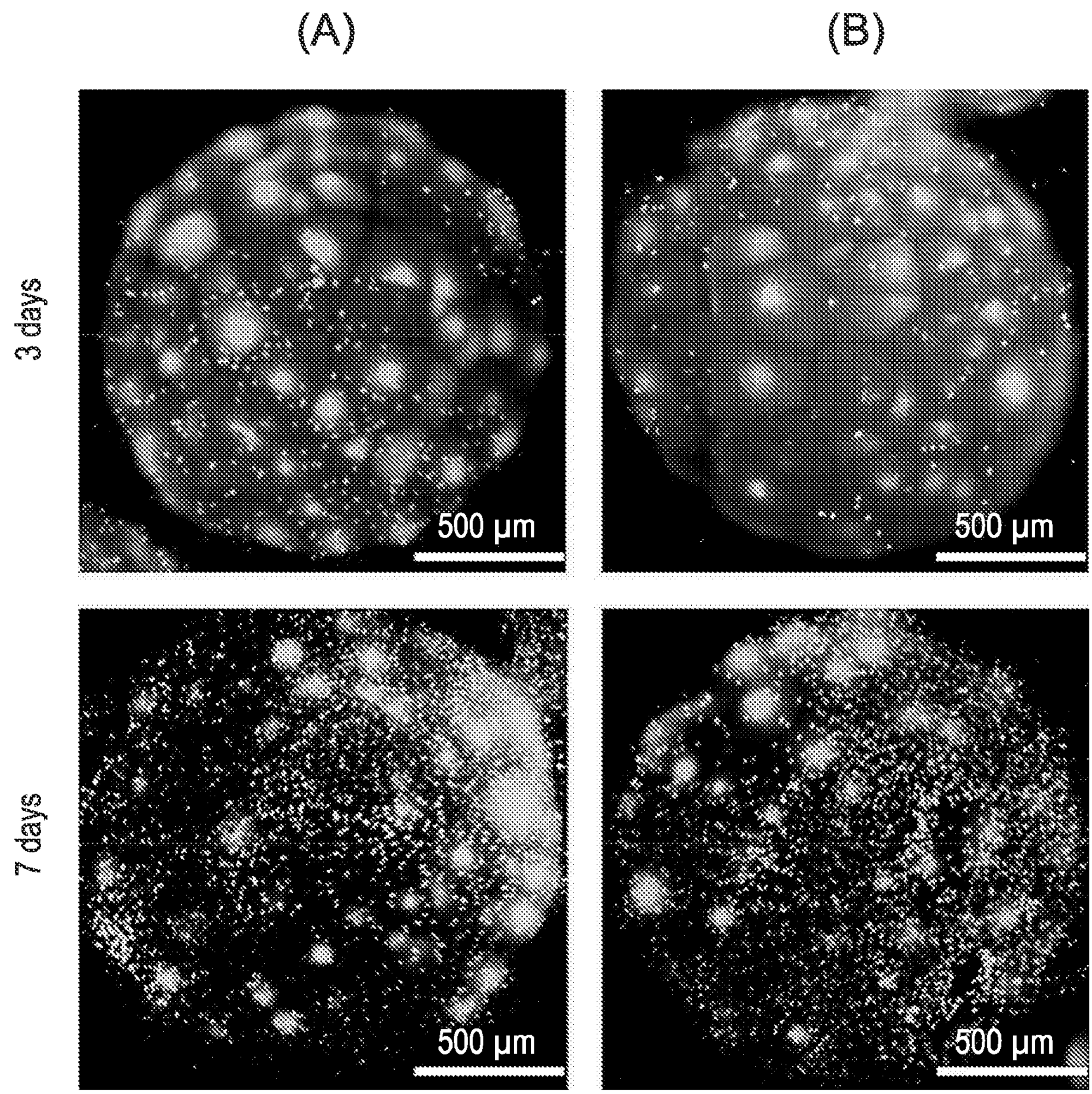


FIG. 13

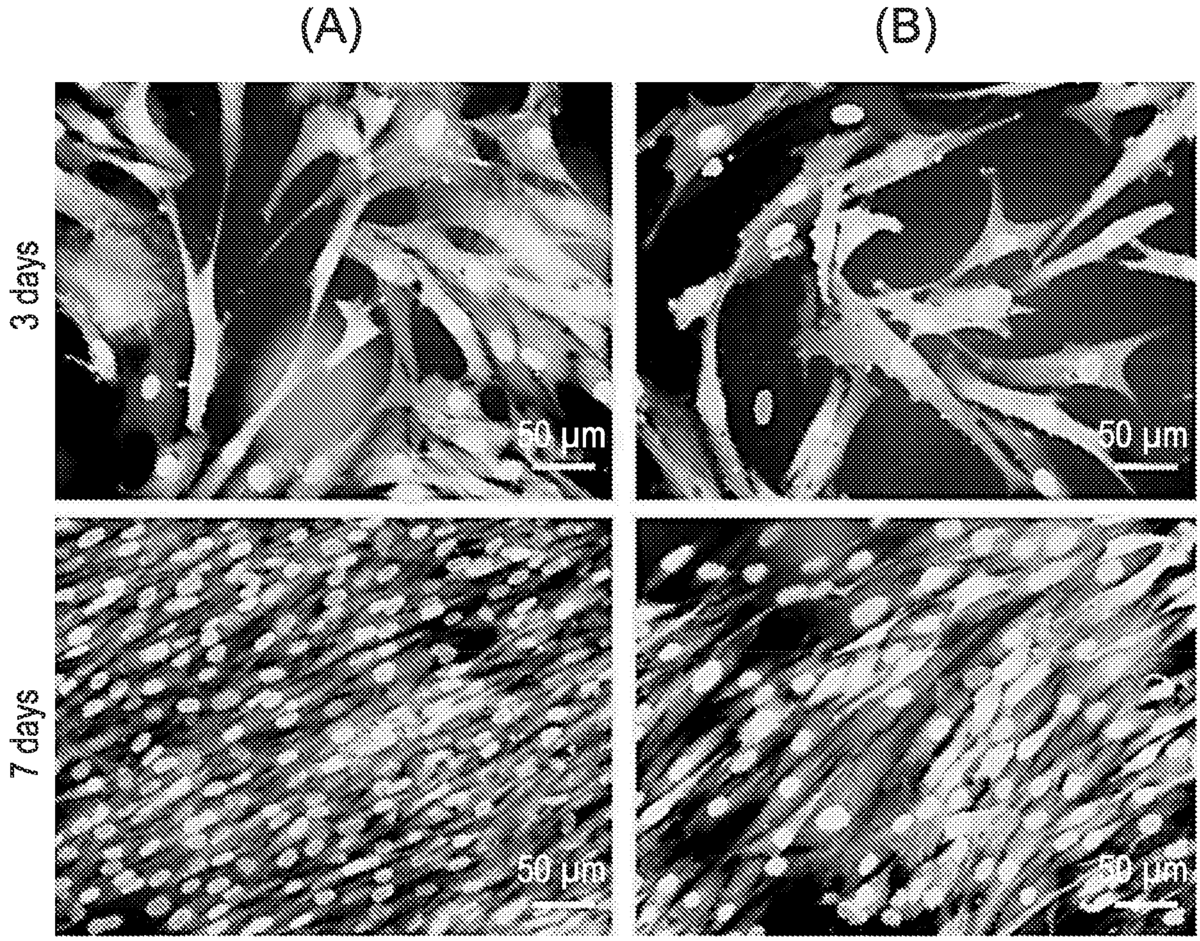


FIG. 14

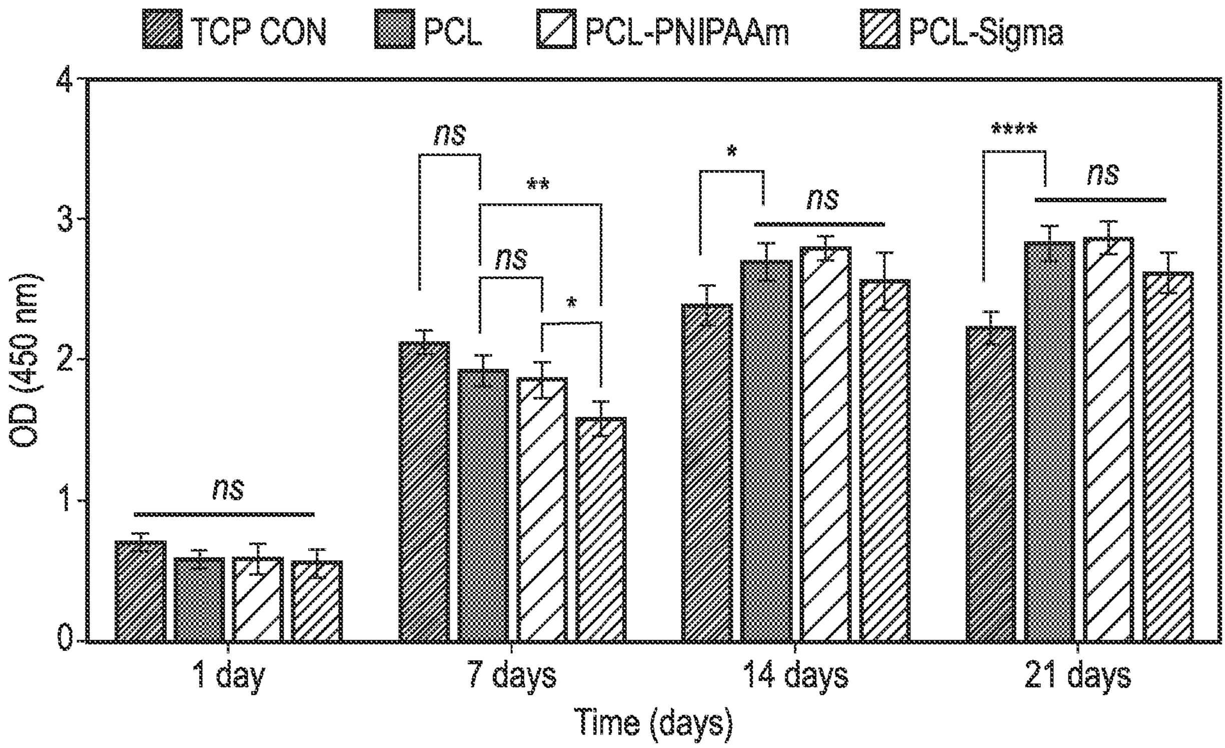
TCP CON
PCL
PCL-PNIPAAm
PCL-Sigma
OD (450 nm)
0
1
2
3
4
ns
ns
**
ns
*
*
ns
****
ns
1 day
7 days
14 days
21 days
Time (days)

FIG. 15

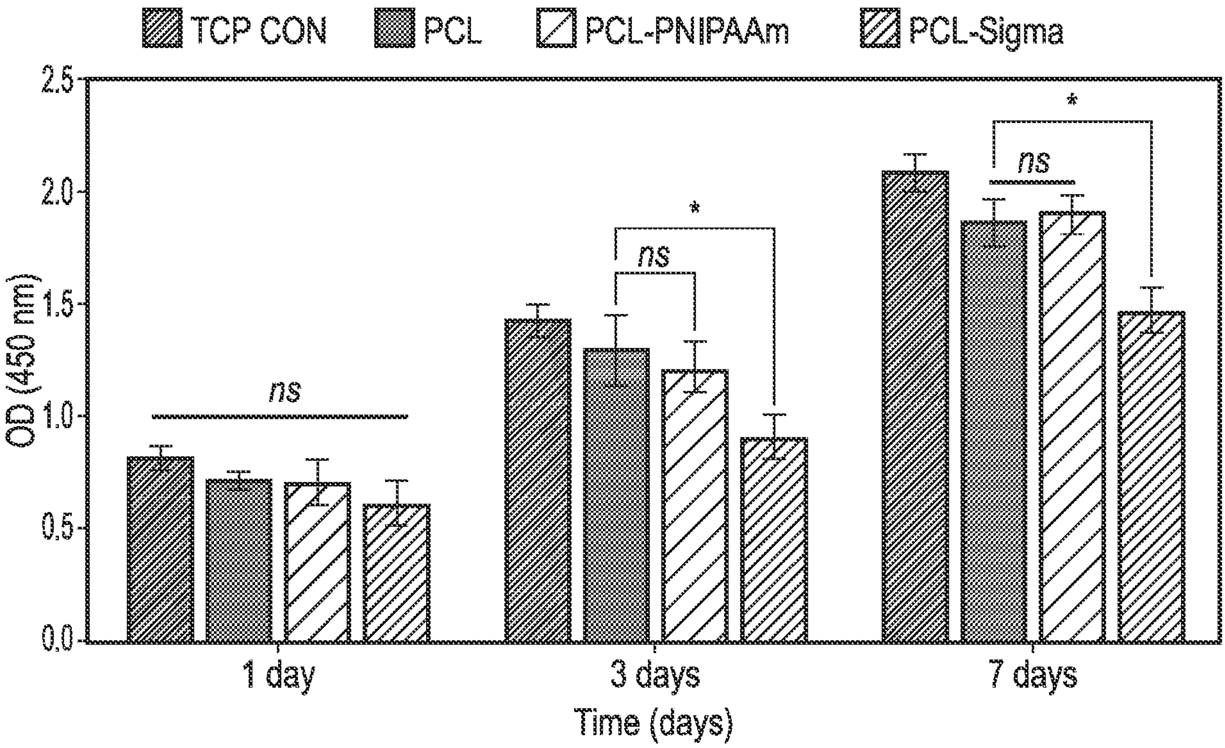
TCP CON
PCL
PCL-PNIPAAm
PCL-Sigma
OD (450 nm)
0.0
0.5
1.0
1.5
2.0
2.5
ns
*
ns
*
ns
1 day
3 days
7 days
Time (days)

FIG. 16

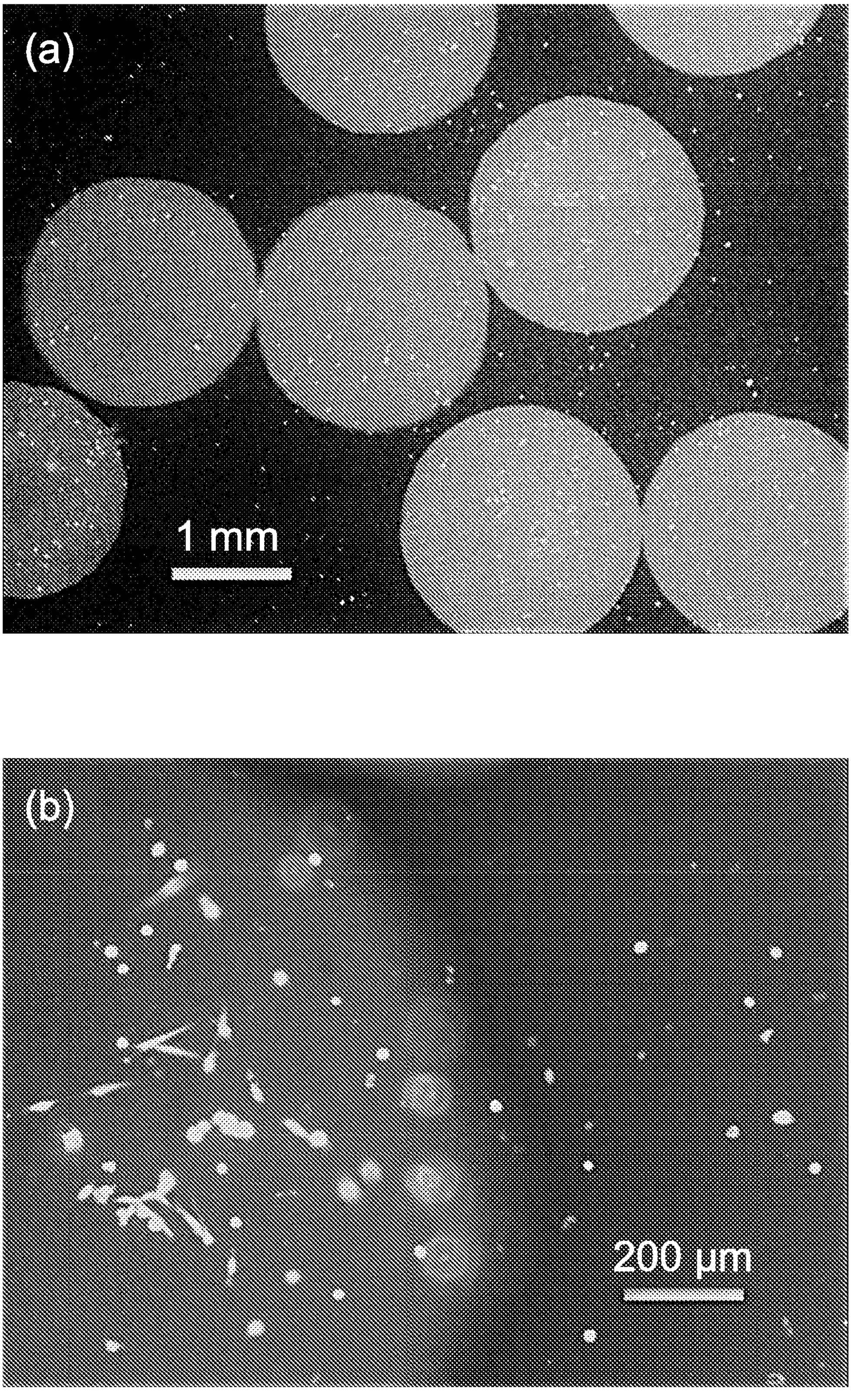


FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014143871 A2
- US 2012156777 A1
- WO 2008005520 A2
- US 2017081638 A1


### Non-patent literature cited in the description


- *Cell Transplantation,* 2010, vol. 19, 1123-1132
- **M. R. KIM et al.** *Biotechnol. Prog.,* 2002, vol. 18, 495-500